(19) Europäisches Patentamt
European Patent Office
Office européen des brevets

(11) **EP 4 212 205 B1**

(12) **EUROPEAN PATENT SPECIFICATION**

(45) Date of publication and mention
of the grant of the patent:
**01.05.2024 Bulletin 2024/18**

(21) Application number: **22382014.3**

(22) Date of filing: **13.01.2022**

(51) International Patent Classification (IPC):
*A61N 1/05* (2006.01)      *A61B 5/24* (2021.01)
*A61N 1/36* (2006.01)      *A61N 1/372* (2006.01)
*A61N 1/375* (2006.01)    *A61N 1/378* (2006.01)
*A61F 2/90* (2013.01)      *A61B 5/00* (2006.01)
*A61B 5/293* (2021.01)    *A61B 5/37* (2021.01)
*A61F 2/82* (2013.01)      *A61B 5/01* (2006.01)
*A61B 5/03* (2006.01)      *A61B 5/145* (2006.01)

(52) Cooperative Patent Classification (CPC):
**A61N 1/37514; A61B 5/0031; A61B 5/293;
A61B 5/37; A61B 5/686; A61B 5/6861;
A61B 5/6868; A61F 2/90; A61N 1/0534;
A61N 1/36053; A61N 1/37205; A61N 1/37516;
A61N 1/3756; A61N 1/3787;** A61B 5/0006; (Cont.)

(54) **SYSTEM FOR POWERING AND CONTROLLING THE ELECTRONICS OF MINIMALLY INVASIVE STIMULATION OR MONITORING DEVICES IMPLANTABLE IN HEAD OR NECK BODY REGIONS**

SYSTEM ZUR VERSORGUNG UND STEUERUNG DER ELEKTRONIK VON IN KOPF- ODER HALSKÖRPERBEREICHEN MINIMALINVASIV IMPLANTIERBAREN STIMULATIONS- ODER ÜBERWACHUNGSVORRICHTUNGEN

SYSTÈME D'ALIMENTATION ET DE COMMANDE DE L'ÉLECTRONIQUE DE DISPOSITIFS DE STIMULATION OU DE SURVEILLANCE MINIMALEMENT INVASIFS IMPLANTABLES DANS LES RÉGIONS CORPORELLES DE LA TÊTE OU DU COU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.07.2023 Bulletin 2023/29**

(73) Proprietor: **Universitat Pompeu Fabra**
**08002 Barcelona (ES)**

(72) Inventors:
• **IVORRA CANO, Antoni**
**08002 Barcelona (ES)**
• **GARCÍA MORENO, Aracelys**
**08002 Barcelona (ES)**
• **TUDELA PI, Marc**
**08002 Barcelona (ES)**

(74) Representative: **TRBL Intellectual Property**
**Glorieta de Quevedo, 8**
**28015 Madrid (ES)**

(56) References cited:
**WO-A2-2013/035092     US-B2- 10 729 530**

• **LAURA BECERRA-FAJARDO ET AL: "Demonstration of 2 mm Thick Microcontrolled Injectable Stimulators Based on Rectification of High Frequency Current Bursts", IEEE TRANSACTIONS ON NEURAL SYSTEMS AND REHABILITATION ENGINEERING, vol. 25, no. 8, 1 August 2017 (2017-08-01) , pages 1343-1352, XP055669120, USA ISSN: 1534-4320, DOI: 10.1109/TNSRE.2016.2623483**
• **ALDAOUD AMMAR ET AL: "Near-Field Wireless Power Transfer to Stent-Based Biomedical Implants", IEEE JOURNAL OF ELECTROMAGNETICS, RF AND MICROWAVES IN MEDICINE AND BIOLOGY, IEEE, vol. 2, no. 3, 1 September 2018 (2018-09-01), pages 193-200, XP011689232, ISSN: 2469-7249, DOI: 10.1109/JERM.2018.2833386 [retrieved on 2018-08-21]**

EP 4 212 205 B1

- **BECERRA-FAJARDO LAURA ET AL: "In Vivo Demonstration of Addressable Microstimulators Powered by Rectification of Epidermically Applied Currents for Miniaturized Neuroprostheses", PLOS ONE, vol. 10, no. 7, 6 July 2015 (2015-07-06), page e0131666, XP055829278, DOI: 10.1371/journal.pone.0131666**

(52) Cooperative Patent Classification (CPC): (Cont.)
A61B 5/01; A61B 5/031; A61B 5/14539;
A61B 2560/0219; A61B 2562/162; A61F 2250/0002

**Description**

**FIELD OF THE INVENTION**

The present invention belongs to the field of medical technologies. More specifically, the invention relates to a system suitable for powering and controlling the electronics of minimally invasive devices implantable in head or neck regions such as, for example, implantable devices configured for modifying or recording nervous activity, wherein the powering and controlling signals exchanged between the system and the electronics of the implantable devices are based on volume conduction. The system enables bidirectional interfacing with the head or neck body region, thereby allowing to record electrical activity therefrom, and/or to provide electrical stimulation thereto.

**BACKGROUND OF THE INVENTION**

Brain-computer interfacing (BCI) enables the control of devices such as robotic limbs, prostheses, or screen cursors through interpretation of the neural activity. For this reason, BCI can be used in different applications such as restoring motor functions to impaired patients or providing neurofeedback. BCI systems are currently explored in multidisciplinary research involving neuroscience and engineering, which typically involves performing the following steps: collecting brain signals, interpreting them, and outputting control commands to one or more controlled devices according to the brain signals received. Brain signals are typically collected in the form of biopotentials, and these are recorded by means of electrodes which can be placed on the surface of the head, on the surface of the cerebral cortex (i.e., within the skull) or within the brain, either within the brain parenchyma or within its vessels. Surface electrodes provide the least level of invasiveness, but intracranial electrode arrangements and electronic implants provide far superior functional features for BCI.

Electronic implants and implanted electrodes are also used to provide therapeutic electrical stimulation. Electrical stimulation therapies are of use for treating a large variety of diseases affecting the nervous system, or other body organs. In the case of the brain, these therapies may involve direct stimulation of the brain (e.g., in deep brain stimulation) or indirect stimulation of the brain (e.g., in vagus nerve stimulation). Deep brain stimulation (DBS) devices apply electrical impulses to certain deep brain regions for modulating brain circuitry behaviour. Nowadays, DBS is a widely accepted technique for treating movement disorders, such as Parkinson disease, tremor, and dystonia. Also, DBS has been used for pain syndromes, such as neuropathic pain and cluster headache, as well as for epilepsy. A favourable safety profile and demonstration of efficacy in several randomized controlled trials has also led to increased interest in the potential application of DBS to psychiatric disorders, like obsessive-compulsive disorder, depression, Alzheimer disease, Tourette syndrome, addiction, anorexia nervosa and schizophrenia. The minimally invasive character of DBS, combined with the low incidence of its adverse effects, has expanded its potential uses on novel applications for conditions such as tinnitus, arterial hypertension, and sleep disorders, as stated in Krauss et al. ["Technology of deep brain stimulation: current status and future directions". Nature Reviews Neurology, 1-13 (2020)].

Current DBS systems comprise a lead or electrical cable of about 1 mm in diameter attached to multiple electrodes in its end. Said lead is tunnelled across the brain and the skull, and then it is subcutaneously connected to an extension lead. This extension lead is subcutaneously tunnelled from the head to an implantable pulse generator (IPG) implanted into a subcutaneous pocket formed below the collarbone. The lead attached to the electrodes is implanted into a target region by means of brain surgery, typically using a stereotactic approach. The IPG is programmed to send electrical pulses to the brain, and can be controlled with an external control unit. The battery life of the IPG varies with usage and settings and, when it is depleted, needs to be surgically replaced.

In DBS systems, complex measurements are performed before their implantation within a brain region, in order to accurately pinpoint the best location for the electrodes. However, after implantation the leads suffer from migration due to relative movements between the skull (where the leads are anchored) and the brain. These relative movements constitute a major problem in DBS systems, as the undesired displacements of the leads dislodge the electrodes from their therapeutic target sites hence causing treatment inefficacy or clinical complications for the patient.

Other problems associated with the leads in DBS systems are the risks of infection and of hardware malfunction due to rupture of the conductors by mechanical fatigue. This is particularly relevant in movement disorders since the leads are subjected to considerable mechanical stresses along the neck. Consequently, the leads of DBS systems may increase the risk of failure of the implant.

DBS systems and most intracranial BCI systems can be considered as parenchymal implants because the electrodes are placed within or in direct contact with the parenchyma of the brain. In the field of implantable devices, there exists an alternative to parenchymal implants: endovascular implants. An example of these devices are the neurovascular stents labelled as stentrodes and which are described in S.E. John et al. ["The future potential of the Stentrode". Expert review of medical devices, 16(10), 841-843 (2019)]. Said stentrodes comprise a miniaturized electrode array and are implanted into a blood vessel in the brain, without the need of open brain surgery. A second stent for sensing and stimulating tissue is disclosed in patent US 10,729,530 B2, which is designed to be implanted within blood vessels (either in the lumen or in the walls). These electrode arrangements are electrically connected with long endovascular leads to electronic units subcutaneously implanted in the collarbone region. The presence of long leads conveys clinical complications and functional limitations. For instance, the number of electrodes in the implant is limited by the wire thickness, which limits the maximum number of independent connections that can be stablished without occluding the vessel. As claimed by some of the authors of US 10,729,530 B2 in the reference T. J. Oxley et al. ["Minimally invasive endovascular stent-electrode array for high-fidelity, chronic recordings of cortical neural activity". Nature biotechnology, 34(3), 320-327 (2016).], a potentially safer alternative to their wired design would involve a wireless signal and power transmission system, but currently available technology remains too large for safe endovascular deposition. In the above referred documents, no indication is provided on how to integrate the electronics with the stent, which is not a trivial step for an expert in the field.

As stated above, the use of leads is a major drawback of these neurovascular stents. The leads pass along the blood vessels from the recording electrodes to the chest, where the electronics are typically placed. The transvascular exit of the leads is a concern that needs to be addressed. For instance, it may render the implantation site unusable for medical monitoring, or it may prevent intravenous injections, and the blood vessel may possibly rupture causing internal bleeding. Such risks undermine the practical advantages of the stent approach in comparison to penetrating electrodes. Therefore, it would be advantageous to miniaturize the electronics and integrate them into the neurovascular stent such that there is no need for any long wires through the vessels. However, scaling down the implant electronics and, in particular, their power supply is not a trivial task. Power supply techniques in use, such as batteries and inductive coupling, hinder developing wireless electronic units small enough to fit through a deployment catheter while also fitting into a cylindrical or irregular blood vessel.

A viable option to further miniaturize electronic implants is the use of wireless power transfer (WPT) based on volume conduction (also referred to as capacitive coupling, see G. L. Barbruni et al. ["Miniaturised Wireless Power Transfer Systems for Neurostimulation: A Review". IEEE Transactions on Biomedical Circuits and Systems (2020)]).

However, brain implants based on volume conduction like the ones disclosed in G. L. Barbruni et al. can only be shallowly implanted, and their structure is relatively flat (thin but with large surface), which implies a more invasive deployment in the human body. It follows therefore that improved shaping configurations for the implants would be advantageous for obtaining minimal invasiveness, while obtaining maximum collectable power.

Volume conduction can be used to reliably transfer power to implants in human or animal limbs intended for neuromuscular stimulation and sensing purposes, as described in J. Minguillón et al. ["Powering electronic implants by high-frequency volume conduction: in human validation". bioRxiv (2021)]. One example in which minimally invasive implants based on volume conduction are developed for neuromuscular stimulation in the limbs is disclosed in L. Becerra-Fajardo et al. ["Demonstration of 2-mm thick microcontrolled injectable stimulators based on rectification of high frequency current bursts", IEEE Transactions on Neural Systems and Rehabilitation Engineering, vol. 25, no. 8, pp. 1343-1352, Aug. 2017]. This document describes a system for powering and controlling the electronics of a rabbit limb implant by volume conduction, by means of external electrodes. However, this document is silent about how these implants and the external devices would be adapted and configured for the head and neck regions.

Thus, a first technical problem to be solved by the present invention is how to find improved configurations for the implants so as to obtain minimal invasiveness, while obtaining maximum collectable power, thereby enabling intraparenchymal brain stimulation or brain activity recording, by means of an improved and optimized shape which allows further miniaturization compared to the known devices.

A further technical problem solved by the invention is how to conveniently shape brain implants so that they can contain or are connected to multiple electrodes, either for recording electrical activity or for performing electrical stimulation.

As a summary, known parenchymal and endovascular implants suffer from limitations about cabling, longevity, and miniaturization. The invention disclosed in this application overcomes said problems by providing a system configured for powering and communicating with the electronics of an implant, which is based on volume conduction, and which allows to conveniently shape the implants to minimize their size, while maximizing the amount of transferred power.

## BRIEF DESCRIPTION OF THE INVENTION

The invention is defined by the independent claim 1. The object of the invention is a system for powering or controlling the electronics of head or neck implants by volume conduction, in a minimally invasive way. In this way, the system of the invention can be generally configured, in a preferred embodiment of the invention, as a nervous system interface comprising one or more implants and an external unit. Within the scope of this invention, the term "interface" will be understood as a system suitable for exchanging signals (i.e., communicating) with the implants arranged at said head or neck body regions, thus including not only the possibility of taking measurements or recording information from such body regions, but also bringing stimulation to them, or enabling both stimulation and recording capabilities simultaneously.

As it has been outlined in the background section, the need of long wire leads in both conventional parenchymal implants (including DBS systems) and endovascular implants is troublesome. For removing the need of wire leads, a technical challenge that this invention addresses is to wirelessly transfer power (by volume conduction) to and communicate with electronic implants within a body region of the head or the neck (e.g., the brain). An additional technical challenge is to be able to shape the implants conveniently so that they are minimally invasive and still contain or are connected to multiple electrodes, either for recording electrical activity or for performing electrical stimulation.

According to the present invention, it is possible to power elongated implants with a thin cross-section which are located in the head or the neck by using an alternating current (ac) of high frequency (HF) delivered by volume conduction. Preferably, said frequency belongs to the interval 1-200 MHz, and more preferably, is between 5 MHz and 20 MHz to comply with safety standards and to prevent significant effect of the skin. The alternating current (ac) is applied more effectively in the form of short bursts rather than continuously, which enables large magnitude HF currents to be safely delivered through the human body.

The implants have preferably an elongated shape in order to facilitate implant deployment by means of tubular instruments such as those used in minimally invasive procedures and to facilitate wireless power transfer and communications via volume conduction.

Two general preferred embodiments are envisioned for the implants of the invention:

1. Parenchymal implants (e.g., a leadless DBS) shaped very similarly to a short (with a length of only a few centimetres) version of current DBS leads but without any sort of tethering or electrical wires.

2. Leadless endovascular implants (e.g., a leadless stentrode) which avoids the need for electrical connections, as the electronics unit of the implant can be integrated together with the neurovascular stent or at a short distance from it, within the body region (e.g., brain, neck, upper thorax).

The proposed interconnection and encapsulation techniques for parenchymal and leadless endovascular implants according to this invention ensure: i) minimal invasiveness, ii) effective coupling based on volume conduction, iii) hermeticity to protect the electronics of the implants, and iv) effective and robust electrical connection with the electrodes for stimulation or for recording.

The implants proposed by the invention are mainly shaped as a slender and semi-flexible elongated bodies This configuration facilitates implant deployment (even in deep regions), anchorage, and extraction. Note that if the cross-section of the device to be implanted within the vessel's lumen is too large, then it is not suitable for its deployment in smaller vessels (i.e., it would occupy an excessive portion of the lumen of the vessel). In this context, it is advantageous that the present invention avoids the use of a flat design for the implant electrodes responsible for coupling based on volume conduction. Even though flat electrodes with a large surface area provide a maximization of power transfer efficiency (particularly in setups where the receiver electrodes are close to the transmitter electrodes), the use of large surface electrodes precludes implantation of the implants via minimally invasive procedures.

In view of the above features, a first object of the invention refers to a system for powering the electronics of nervous system implants by volume conduction, comprising:

- at least one elongated implant, adapted to be implanted inside a body region, said body region being comprised in a human or animal head or neck, wherein the implant comprises:

  a) a hermetic capsule;
  b) a plurality of primary electrodes, comprising a volume conduction electrode pair; said primary electrodes being arranged, at least in part, externally to the capsule;
  c) an electronic circuit housed inside the capsule and electrically connected to the primary electrodes, comprising:

  - a digital control unit;
  - a power transfer stage adapted to receive at least part of the energy of a high-frequency current transmitted by volume conduction through the volume conduction electrode pair, and to transfer the power associated to said at least part of the energy of the high-frequency current to the digital control unit and to a communications stage;
  - a communications stage adapted to detect, across two of the primary electrodes, the high-frequency current transmitted by volume conduction to the volume conduction electrode pair; and to process the high-frequency current by means of the digital control unit; and

- an external unit adapted to generate at least the high-frequency current to transfer power to and to communicate with the at least one implant; wherein the fundamental frequency of the high-frequency current is comprised between 1 MHz and 200 MHz.

Advantageously in the invention, the external unit is electrically connected to a plurality of external electrodes, wherein at least two of said external electrodes comprise corresponding contact areas adapted to be arranged over the body region, at substantially opposite positions along a longitudinal axis defined by the elongated implant; and wherein the sum of contact areas of each said external electrodes is comprised between 1 cm$^2$ and 250 cm$^2$.

In a preferred embodiment of the invention, the primary electrodes of the implant further comprise a plurality of communication electrodes; and the communications stage of the electronic circuit of the implant is further adapted to exchange, through two of the primary electrodes, one or more management signals with the external unit by volume conduction.

In a further preferred embodiment of the invention, the electronic circuit of the implant further comprises a biopotentials acquisition stage comprising amplifiers, band-pass filters, analog-to-digital converters and switching elements, said biopotentials acquisition stage being adapted to record one or more electrical signals from the body region through at least the volume conduction electrode pair and/or one or more recording and stimulation electrodes, being the electrical signals dependent on a voltage difference outside the capsule.

In a further preferred embodiment of the invention, the electronic circuit of the implant further comprises a neurostimulation stage adapted to generate and deliver electrical signals outside the implant through the volume conduction electrode pair and/or the one or more recording and stimulation electrodes, said neurostimulation stage comprising at least one of the following: a pulse generator, rectification elements and switching elements.

The hermetic capsule of the implant is elongated and comprises one or more dielectric segments interspersed with one or more conductive segments. More preferably, one or more conductive segments of the hermetic capsule are electrically connected to one or more primary electrodes of the implant or to one or more recording and/or stimulation electrodes of the implant, arranged externally of the capsule, by means of one or more wires; said implant comprising a non-hermetic insulation which houses, at least in part, the capsule and the wires.

In a further preferred embodiment of the invention, the hermetic capsule further comprises an internal tubbing to reinforce the structure and, optionally, to house the wires which connect the conductive segments to the electronic circuit.

In a further preferred embodiment of the invention, the conductive segments do not encircle the entire cross-section of the capsule.

In a further preferred embodiment of the invention, the capsule comprises stepped segments in which dielectric segments are interspersed with conductive segments.

In a further preferred embodiment of the invention, the implant is shaped as a semi-flexible threadlike device and wherein the hermetic capsule is contained within a non-hermetic flexible body made of insulating material that also contains the wires to connect the capsule to one or more primary electrodes of the implant or to one or more recording and/or stimulation electrodes of the implant.

In a further preferred embodiment of the invention, the implant comprises a stent-like structure adapted to secure the position of the whole implant against a vessel wall, and wherein the capsule is externally fastened to the stent-like structure by means of one or more fasteners.

In a further preferred embodiment of the invention, the implant is planar and comprises a dielectric substrate made of a flexible or conformable material, and wherein the implant is optionally arranged in contact with a stent-like structure adapted to secure the position of the whole implant against a vessel wall.

In a further preferred embodiment of the invention, the implant comprises an elongated non-rigid structure which can be expanded and secured to a vessel wall by means of the stent-like structure, wherein the implant further comprises a hollow network of connections between the recording and/or stimulation electrodes and the capsule by means of wires which are flexible or plastically deformable.

In a further preferred embodiment of the invention:

- the power transferring stage of the electronic circuit comprises a blocking capacitor connected in series with the volume conduction electrode pair or the plurality of communication electrodes, said blocking capacitor being adapted to prevent passage of dc current and to enable the flow of the high-frequency current to and from the electronic circuit;
- the communications stage comprises a demodulator unit adapted to demodulate the high-frequency current; and
- the digital control unit is connected to a load modulator adapted to modulate symmetrically or asymmetrically the load of the implant.

In a further preferred embodiment of the invention, the external unit comprises:

- a power supply;

- a digital control unit and a modulator adapted to generate high-frequency signals adapted to power and communicate with one or more implants by volume conduction;
- receiving means adapted to receive the one or more signals from the one or more implants through the body region by volume conduction;
- processing means adapted to process information associated to the one or more signals received from the one or more implants; and
- a signal amplifier adapted to amplify the one or more management signals generated by the digital control unit and the modulator; and to deliver said signals to one or more implants through the body region by volume conduction.

In a further preferred embodiment of the invention, the external unit comprises a plurality of external electrodes, and one or more switching elements or permanent interconnections electrically connected to the one or more external electrodes to selectively select a set of them for delivering the high-frequency current and/or to selectively short circuit one or more of them.

In a further preferred embodiment of the invention, the external unit comprises at least one external electrode adapted to be placed on the scalp; and at least one external electrode adapted to be placed at the neck region.

In the context of this invention, three types of electrodes are thus introduced:

- Primary electrodes, which comprise a "volume conduction electrode pair", included in the implants and responsible for picking up power by volume conduction; along with a plurality of communication electrodes which serves to enable communication with external devices. The communication electrodes are optional, so when they are not present in the invention, their functions are carried out by the volume conduction electrode pair.
- A plurality of "recording and/or stimulation electrodes", which can be also included in the implants, serving as auxiliary electrodes in applications such as brain stimulation, wherein the use of the volume conduction pair for delivering stimulation pulses would not provide enough selectivity or spatial resolution.
- A plurality of "external electrodes", placed over a certain region (neck, skull, etc.), and adapted to be connected to an external unit.

Finally, in the context of this invention an "implant" would primarily refer to an implant suitable for being deployed in the brain or neck. However, these applications are not limiting for the scope of the invention.

## BRIEF DESCRIPTION OF THE DRAWINGS

To complete the description and in order to provide for a better understanding of the invention, a set of drawings is enclosed. Said drawings form an integral part of the description and illustrate different preferred embodiments of the invention, which should not be interpreted as restricting its scope of interpretation, but just as an example of how the invention can be carried out.

Figure 1 shows a schematic embodiment of the system according to the present invention.

Figure 2 illustrates an embodiment of the electronic circuit of an implant comprised in a system according to the present invention.

Figure 3 illustrates in greater detail the electronic circuit of an implant comprised in a system according to the present invention.

Figure 4 shows a schematic embodiment of an implant comprised in a system according to the present invention, wherein the electronic circuit of said implant is housed within a hermetic capsule.

Figure 5 illustrates the electronics of an external unit of the system of the invention, in a preferred embodiment thereof, which provides the high-frequency current to feed one or more implants.

Figures 6A-B display two capsule embodiments for an implant comprised in the system according to the present invention.

Figure 7 shows the structure of a capsule engineered for an implant comprised in a system according to the present invention.

Figure 8 illustrates a capsule for an implant comprised in the system according to the present invention, wherein conductive segments do not encompass the entire perimeter of the capsule.

Figure 9 illustrates an embodiment of the arrangement of the implant electronics and its connections within a capsule for an implant comprised in a system according to the present invention.

Figure 10 shows a capsule for an implant comprised in the system according to the present invention, wherein the dielectric segment is a single continuum.

Figure 11 illustrates an implant comprised in a system according to the present invention, wherein the capsule is contained within a semi-flexible body.

Figure 12 shows an implant comprised in a system according to the present invention that can be secured within a blood vessel.

Figure 13 shows an implant comprised in a system according to the present invention that can be secured within a blood vessel by using a hollow cylindrical mesh.

Figure 14 illustrates the electrical connections required for the same implant displayed in Figure 13.

Figure 15 shows how to use multiple fixing structures, for instance two stent-like segments, to form an implant comprised in a system according to the present invention.

Figure 16 illustrates another implant configuration according to the present invention, wherein stent-like segments are electrically connected to operate as electrodes of the implant comprised in a system of the invention.

Figure 17 shows a configuration for an implant comprised in a system according to the present invention, wherein the capsule is secured in-between a stent-like fixing structure and a blood vessel wall.

Figures 18-19 show two configurations for a system according to the present invention, wherein the implant is attached to a stent-like structure but in which the stent-like structure is not part of the implant itself.

Figure 20 shows external electrode arrangements with two electrodes, which are suitable for systems according to the present invention.

Figure 21 shows an external electrode arrangement for a system according to the present invention, comprising two implants with predominant orientations perpendicular to each other, an external unit and switching elements.

Figure 22 shows an external electrode arrangement for a system according to the present invention, suitable to be placed on the neck or on the upper thorax. This arrangement is appropriate to induce electric fields for powering elongated brain implants predominantly oriented in the vertical direction in relation to the human head.

Figure 23 shows another external electrode arrangement for a system according to the present invention, suitable to be deployed in the head.

Figure 24 shows an external electrode arrangement for a system according to the present invention, suitable for stimulation of the vagus nerve.

Figure 25 illustrates the concept of virtual electrode formation for a system according to the present invention.

Figure 26 shows how to attach the external electrodes of a system according to the present invention, by using electrically passive securing structures, such as headset or headbands.

Figure 27 illustrates the geometrical and anatomical head model applied to numerically simulate the performance of a system according to the present invention in the brain region. Each colour corresponds with a different tissue: muscle tissue (in black), skull (in white), grey matter (in dark grey) and white matter (in light grey). Two different views are shown: a) Frontal plane. b) Midsagittal plane.

Figure 28 shows the simulation results of a system according to the present invention, which comprises an implant deployed in the brain parenchyma and three external electrodes: two of them are placed below the neck and the third one is located at the top of the head. Figure 28A shows the maximum current that can be applied through the external electrodes, while Figure28B shows the maximum power that the implant can collect, without exceeding the SAR limit of 10 W/kg.

Figure 29 shows the simulation results for a system according to the present invention, comprising a three-electrode vertical arrangement, with an external electrode radius of 3 cm.

Figure 30 shows the simulation results for a system according to the present invention, comprising a three-electrode vertical arrangement, with an external electrode radius of 1 cm.

Figure 31 summarizes the simulation results for a system according to the present invention, comprising a three-electrode horizontal arrangement.

Figure 32 displays a two-electrode arrangement for a system according to the present invention, with a single top external electrode placed at the top of the parietal region, and a single external electrode placed at the top of the frontal region.

Figure 33 shows the considered geometrical and anatomical model of the neck region considered for simulating a system according to the present invention. Two views are displayed: a) Frontal plane including the volume conduction electrode pair. b) Symmetrical sagittal plane including the volume conduction electrode pair. The external electrodes (not shown) consist of two disks on the surface of the neck aligned with the implant electrodes.

Figure 34 shows the maximum power attainable in an implant comprised in a system according to the present invention, when the implant is placed at the jugular vein. The maximum local SAR values do not exceed 10 W/Kg. The geometrical configuration is as follows: electrode diameter (D= 1 mm), length (2 mm), separation between electrodes (S= 30 mm), and separation with the cylinder blood wall (0.5 mm).

In these figures, the following reference numbers are used:

| | |
|---|---|
| 1, 1' | Implants |
| 2 | Body region |
| 3 | Hermetic capsule |
| 3' | Dielectric segments of the capsule |
| 3" | Conductive segments of the capsule |
| 4, 4' | Volume conduction electrode pair |
| 5 | Electronic circuit of the implant |
| 6, 6' | Digital control units |
| 7 | Power transfer stage |
| 7' | Rectifier |
| 7" | Voltage regulator |
| 7"' | Blocking capacitor |
| 7"" | Storage capacitor |
| 8 | Communications stage |
| 9 | External unit |
| 10, 10', 10", 10"' | External electrodes |
| 11, 11', 11", 11"' | Recording and/or stimulation electrodes |
| 12, 12' | Demodulator |
| 13 | Biopotentials acquisition stage |

(continued)

| | |
|---|---|
| 14 | Neurostimulation stage |
| 15 | Switching elements |
| 16 | Pulse generator |
| 17 | Load modulator |
| 18, 18' | Signal amplifier |
| 19 | Hermetic electrical connections |
| 20 | Wires insulation |
| 21 | Power supply |
| 22 | Modulator |
| 23 | Current sensor |
| 24 | Wires |
| 24' | Multiwire cable |
| 25 | Non-hermetic insulation |
| 26 | Metal ring |
| 27 | Metal cylinder |
| 28 | Tubbing |
| 29 | Substrate |
| 30 | Electronic components |
| 31 | Conductive elements |
| 32 | Non-hermetic flexible body |
| 33 | Blood vessel |
| 34, 34' | Fixation elements |
| 34", 34"' | Stent-like segments of the fixation element |
| 35 | Fastener |
| 36 | Virtual electrode |
| 37 | Electrically passive securing structure |
| 38 | Muscle |
| 39 | Skull bone |
| 40 | Brain white matter |
| 41 | Brain grey matter |

## DETAILED DESCRIPTION OF THE INVENTION

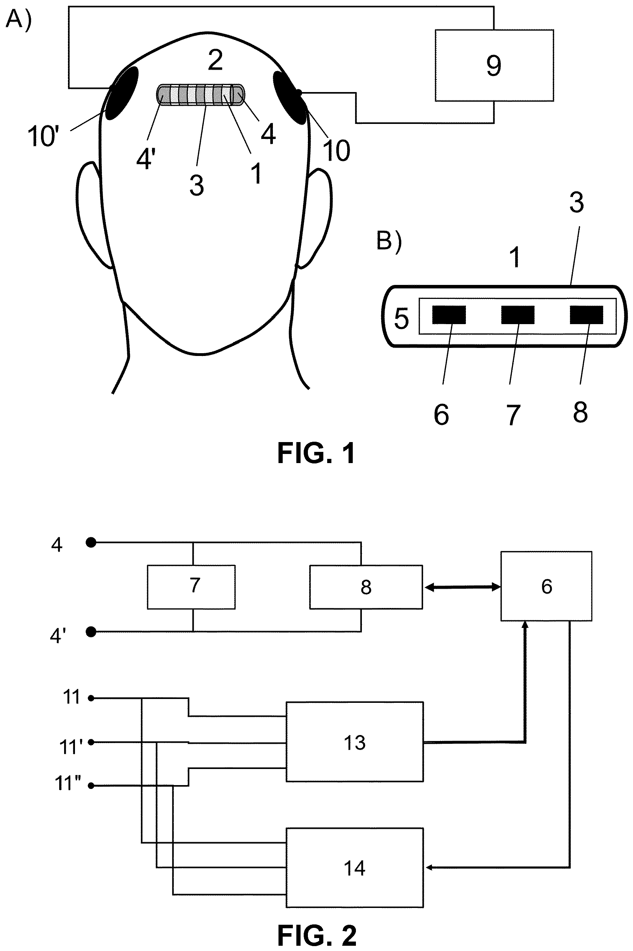

Different embodiments for the system of the invention, which are suitable for powering or controlling the electronics of implantable devices adapted for stimulation or monitorization, will be now described for illustrative purposes. The system, as illustrated in Figures 1A-1B, comprises at least one elongated implant (1), adapted to be implanted inside the head or neck body region (2), wherein said body region (2) comprises a human or animal head or neck. More specifically, the implant (1) of the device comprises:

-    a hermetic capsule (3);
-    a plurality of primary electrodes comprising a volume conduction electrode pair (4, 4') and optionally, a plurality of communication electrodes, wherein said primary electrodes are arranged, at least in part, externally to the capsule (3); and

- an electronic circuit (5) housed inside the capsule (3) and electrically connected to the primary electrodes.

In a preferred embodiment of the invention, the electronic circuit (5) of the implant (1) comprises, in turn:

- a digital control unit (6), abbreviated as DCU. The DCU (6) is configured to receive, process and/or deliver electrical signals.
- a power transfer stage (7) configured to receive at least part of the energy of a high-frequency current transmitted by volume conduction through the volume conduction electrode pair (4, 4'), and to transfer the power associated to said energy to the digital control unit (6) and to a communications stage (8) of the electronic circuit (5).
- a communications stage (8) adapted to detect, across two of the primary electrodes, either the volume conduction electrode pair (4, 4') or the communication electrodes, the high-frequency current transmitted by volume conduction to the volume conduction electrode pair (4, 4'); and to process the signal with the digital control unit (6).

The system of the invention further comprises an external unit (9) adapted to generate at least the high-frequency current to transfer power to and to communicate with the at least one implant (1). Preferably, the fundamental frequency of the high-frequency current is comprised between 1 MHz and 200 MHz.

Advantageously in the invention, the external unit (9) is electrically connected to one or more external electrodes (10, 10'), for instance arranged in pairs. At least two of said external electrodes (10, 10') comprise corresponding contact areas adapted to be arranged over the head or neck body region (2) at substantially opposite positions along an axis defined by the elongated implant (1). The external unit (9) may be placed outside the human or animal body, or also implanted subcutaneously (at least in part) but outside the implant (1). More preferably, the sum of contact areas for each electrode is comprised between 1 cm$^2$ and 250 cm$^2$. The technical effect associated with these features is enabling a sufficient power collection. For this purpose, both a large contact area of the external electrodes (10, 10') along with the alignment of the implant (1) are required.

Figure 1A shows a general architecture for the system of the invention, placed in the brain region. Figure 1B shows a close-up view of the implant (1) with its main components. In this embodiment, the hermetic capsule (3) also serves as an external housing for the implant (1), thereby protecting the electronic circuit (5) and enabling a long-term implantation. Possible uses of the invention comprise recording and stimulating brain tissues, and also the vagus nerve by implanting them in the jugular vein at the neck.

In addition, or alternatively to communications via volume conduction, communications can be performed via other known methods for communications with implants (1) such as far-field RF transmission with antennas, near-field transmission with coils or optical transmission with optoelectronic elements. These methods can result in bulkier implants as they imply embedding additional parts in the implants (1), like the coils. However, they may provide advantages such as transmission at longer distances or higher transmission speeds.

The DCU (6) of the implant (1) can comprise, for instance, a computer processing unit (CPU), a field-programmable gate array (FPGA) or a general digital microprocessor equipped with processing means to record and process electrical signals from nervous system activity. The DCU (6) manages actions typical of a signal acquisition system and/or of a stimulation system. The DCU (6) either executes an algorithm or interprets data and commands sent by the external unit (9) and, in response to those, executes preprogramed routines. The routines can comprise recording the biopotentials for a limited period, processing such recordings (for instance, by obtaining the root-means-square voltage of the signal recordings) and sending the result back to the external unit (9).

Volume conduction is suitable for powering the implants (1) and for enabling bidirectional digital communications between the implant (1) and the external unit (9). The DCU (6) of the implant (1) can establish a communication with the external unit (9) to exchange measurements and management signals by radio communication or other means. In order to meet the requirements of safety standards and to be able to operate deep implants (1), the use of volume conducted bursts of alternating (ac) currents at frequencies in the range from 1 MHz to 200 MHz is preferable. Such currents are provided by the external unit (9). In this way, volume conduction avoids the need of any bulky component within the implant (1) for power supply.

Figures 2-3 display a possible general architecture for the electronic circuit (5) of the implant (1), which is fed by the power extracted by the power transfer stage (7). The power transfer stage (7) extracts power by collecting, by means of the volume conduction electrode pair (4, 4'), a portion of the high-frequency current that flows through the body region (2), for example by volume conduction, and which is generated by the external unit (9). The volume conduction electrode pair (4, 4') is preferentially located at the opposite ends of the implant (1) for maximizing the power that can be extracted. Furthermore, the implant (1) may comprise other recording and/or stimulation electrodes (11, 11', 11", 11''') to connect additional circuitry and to provide a spatial selectivity on which portions of the body region (2) are excited or measured. In further embodiments of the invention, the implant (1) may comprise other electronic, electromechanical, and/or electrochemical devices connected to the electronic circuit (5) and configured with sensing capabilities, such as pressure sensing, temperature sensing, pH sensing, etc.

The invention comprises different communication schemes, which can be either unidirectional (only from the external unit (9) towards the implant (1), or only from the implant (1) to the external control unit (9)), or bidirectional (enabling an exchange of information between the external unit (9) and the implant (1)). Different methods are possible for implementing the so-called physical layer for both types of communications:

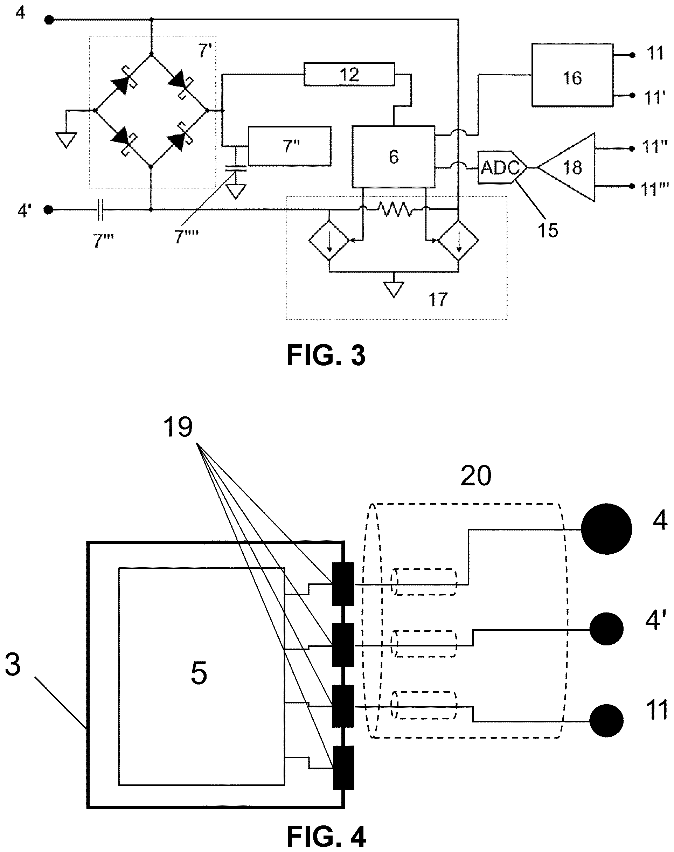

a) Downlink communications from the external unit (9) to the implant (1). The high-frequency current (field) generated by the external unit (9) can be modulated using amplitude-shift key (ASK), frequency-shift key (FSK) and phase-shift key (PSK), among others, to transmit data from the external unit (9) to the implant (1). After such a modulation, the high-frequency current thus becomes a modulated management signal. By picking up the modulated signal across the volume conduction electrode pair (4, 4'), the implant (1) can recover the transmitted data, for example by means of a demodulator (12) (Figure 3). Since the main purpose of the high-frequency current generated by the external unit (9) is to transfer power to the implant (1), it may be convenient to employ methods that minimize impact on power transfer. For instance, if ASK is employed, then Manchester codification is convenient as it provides:

- self-clocking capabilities which are beneficial for demodulation; and
- a constant average amplitude of the signal, ensuring power is being transferred.

Alternatively, downlink communications can be performed in bursts different from those for power transfer, or can be performed only during a fraction of the power transfer burst. During said communications bursts or fractions of power transfer burst, the powering of the implant (1) electronics can be sustained by means of a capacitive element included in the power transfer stage (7), as it is commonly performed in voltage regulators.

b) Uplink communications from the implant (1) to the external unit (9). Two general methods are envisioned to perform data transmission in uplink communications:

- Direct generation, based on known signal generation and modulation methods. In this way, the implant (1) generates a voltage or current signal which is applied through the volume conduction electrode pair (4, 4') thus generating an additional electric field that is transmitted by volume conduction through the surroundings of the body region (2), e.g., living tissues. This additional electric field is then picked up by the external unit (9) electrodes, which recover the transmitted data, for example by means of the demodulator (12).

- Load modulation, in which the implant (1) modulates the electrical load it exhibits for the high-frequency currents, thus producing a management signal. This can be accomplished, for instance, by means of switches (e.g., transistors) that partially short-circuit two electrodes of the implant (1), thus creating an amplitude modulated high-frequency electric field around the implant (1). Then, the external control unit (9) detects the modulated signal by means of known current sensing methods, for instance, by picking up the voltage across a resistor connected in series to the volume conduction electrode pair (4, 4') that deliver the high-frequency current.

The external unit (9) may establish communications with multiple implants (1) in a coordinated manner using known protocols. The signal transmitted by the external unit (9) can encode the address of the implant (1) to which it is sent, either a physical or a logical address (i.e., MAC or IP address). This provides a selective mechanism of interrogating or commanding different implants (1) with the same external unit (9). The communication and addressing protocols can be customized to minimize the duration of the communications, as well as power loss during said communications.

The communications stage (8) is preferably connected to electrodes located at the opposite ends of the implant (1) for maximizing the signal amplitude, and hence the signal to noise ratio, both in the downlink and in the uplink cases. Preferably, these electrodes comprise the volume conduction electrode pair (4, 4') for minimizing the number of electrodes and interconnections, as shown in Figure 2. Alternative embodiments further comprise one or more communication electrodes different from the volume conduction electrode pair (4, 4').

Depending on the intended use, the electronic circuit (5) of the implant (1) can either contain a biopotentials acquisition stage (13), a neurostimulation stage (14), or both.

On the one hand, the biopotentials acquisition stage (13) shown in Figure 2 comprises electronic elements (15) like amplifiers, band-pass filters, analog-to-digital converters (ADC) and switching (or multiplexing) elements, adapted to acquire feeble activity electrical signals from living tissues. The biopotentials acquisition stage (13) is adapted to record one or more electrical activity signals from the body region through the volume conduction electrode pair (4, 4') of the implant (1), being the electrical activity signals dependent on a voltage difference outside the capsule (3). The biopotentials acquisition stage (13) can be implemented either in a single-ended configuration or in a differential configuration with one of the recordings and/or stimulation electrodes (11, 11', 11") defining a ground reference. Preferably, the recording and/or stimulation electrodes (11, 11', 11") used for biopotentials acquisition are different from the volume conduction electrode pair (4, 4').

On the other hand, the neurostimulation stage (14) (see also Figure 2) is adapted to generate and deliver electrical signals outside the implant (1) through the recording and/or stimulation electrodes (11, 11', 11"). The volume conduction electrode pair (4, 4') may also play the role of the recording and/or stimulation electrodes (11, 11', 11 "). Two different architectures are envisioned for this neurostimulation stage (14):

- A first approach comprises the integration of a conventional pulse generator (16) (Figure 3) in the implant (1). Known circuits can be applied to implement the conventional pulse generator (16), for example, an electronic generator able to generate monophasic or biphasic voltage or current pulses with a repetition frequency ranging from 0 Hz (single pulse) to 10 kHz, with a duration ranging from 10 $\mu$s to 10 ms and with amplitudes ranging from 0.1 $\mu$A to 100 mA or from 10 mV to 100 V. As the other sub-circuits of the implant (1), the pulse generator is powered by the power transfer stage. The pulse generator (16) delivers stimulation pulses either through the same pair of electrodes used for power (the volume conduction electrode pair (4, 4')) or other recording and/or stimulation electrodes (11, 11').
- A second approach comprises developing implants (1) working as controllable rectifiers of the volume conducted high-frequency current bursts. By rectifying the high-frequency current bursts across the implant (1), low frequency currents suitable for inducing local stimulation in the head or neck body region (2) are generated. In the implants configured to perform neurostimulation according to this approach, the digital control unit (6) controls the flow of rectified current through the implant (1) by using diodes and transistors. The digital control unit (6) is powered by wireless power transfer (WPT) based on volume conduction. It must be noted, however, that the power used for stimulation does not originate from the implant (1) but directly from the passive transformation of the high-frequency current bursts that flow through the tissues where the implant (1) is located. In this regard, this approach may be advantageous for miniaturization as it avoids the need of a big capacitor to transitorily store the energy for the pulses and avoids the need for low to high voltage converters for the pulses. This stimulation by rectification approach could be useful when high voltages are required for stimulation of certain body regions (2).

Both the absolute maximum attainable power and the maximum attainable dc power exhibit a maximum for a specific load resistance (i.e., optimum load resistance). If the ac field is delivered by the external unit (9) in the form of bursts, it is possible to set the value of the optimum load resistance by adjusting the duty cycle of the bursts.

Figure 3 specifically illustrates in greater detail a preferred embodiment of the electronic circuit (5) of the implant (1), wherein said implant (1) comprises neurostimulation and/or monitoring capabilities. The volume conduction electrode pair (4, 4') picks up a part of the high-frequency current of the interrogation signal flowing across tissues for powering the circuitry. Preferably, the volume conduction electrode pair (4, 4') corresponds to the most separated electrodes of the implant (1). Then, the electronic circuit (5) of the implant (1) comprises:

- A power transfer stage (7) (Figures 1-2) including a diode bridge full-wave rectifier (7') and a voltage regulator (7") in order to transform the HF current into direct current (dc) voltage to power the rest of the circuitry (see these elements in Figure 3). A blocking capacitor (7''') is connected in series with the electrode pair (4, 4') to prevent passage of dc and very low frequency currents and to enable the flow of high-frequency current to and from the electronic circuit (5). Said very low frequency currents, essentially dc, would cause electrochemical reactions at the volume conduction electrode pair (4, 4') and could damage both the surrounding living tissues and the volume conduction electrode pair (4, 4'). In this way, this electronic circuit (5) performs the rectification of high-frequency ac current bursts. Another capacitor (7'''') serves for powering the electronics in-between high-frequency current bursts.

- The demodulator (12) performs the demodulation of a signal embedded in the ac current that is used to power the implant (1). The signal may contain additional information, such as the address of the implant with which the external control unit wants to communicate, or commands for stimulating a target body region.

- A load modulator (17), which is adapted to modulate symmetrically or asymmetrically the load of the implant (1). Said loading can be either used to perform electrical stimulation across the electrode pair (4, 4') when loading is asymmetric (i.e., when seen from the electrode pair the implant behaves as an element of asymmetric conductance), or to perform uplink communications, preferably when loading is symmetric to prevent unsought stimulation.

- The DCU (6), which refers, without exclusion, to a computer processing unit (CPU), a field-programmable gate array (FPGA) or any equivalent general digital microprocessor equipped with processing means, adapted to record and/or process the signals collected by the implant (1), as well as controlling the communication with the neurostimulator stage (14) (Figure 2), in case that the implant (1) is equipped with it.

- A pulse generator (16) connected to the DCU (6), which is adapted to deliver pulses through the recording and/or stimulation electrodes (11, 11') to the implant (1). The neurostimulation stage (14) of Figure 2 can either correspond to this pulse generator (16) or to the load modulator (17) with asymmetric loading, or to a combination of both elements.

- An analogic to digital converter (ADC) (15), connected to the DCU (6) and to a signal amplifier (18), which in turn is connected to the recording and/or stimulation electrodes (11, 11') to the implant (1). Alternatively, the signal amplifier (18) can be connected to the volume conduction electrode pair (4, 4').

In Figure 4, the electronic circuit (5) is housed within the hermetic capsule (3). The implant (1) comprises different electrodes (4, 4', 11) which, preferably, are electrically connected to hermetic electrical connections (19) via conductor wires which are covered with an insulating coating (20). The hermetic electrical connections (19) of the capsule (3) may also act as electrodes (4, 4', 11).

The implant (1) of the system has preferably an elongated shape, in order to facilitate its deployment by means of tubular instruments, such as those used in minimally invasive procedures and to facilitate wireless power transfer and communications, for example via volume conduction.

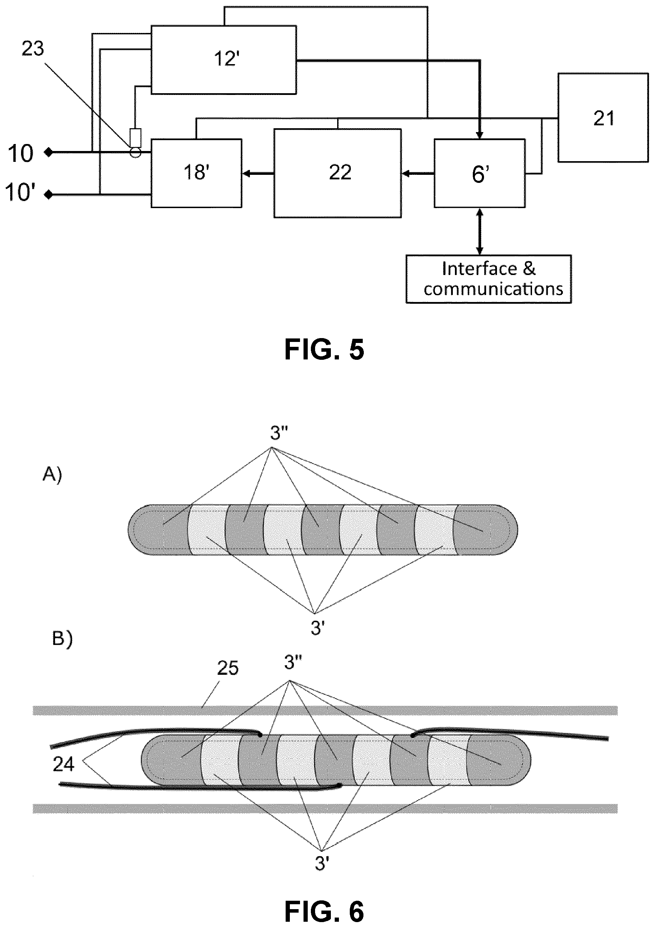

Regarding the electronics of the external unit (9), which provides the high-frequency current, a possible embodiment thereof is depicted in Figure 5. All the circuitry displayed is powered by a power supply (21) which, in turn, preferably comprises a battery or other feeding means. A demodulator (12') is also comprised in the external unit (9), including the required connections for simultaneously implementing the two uplink communication methods previously defined (the implementation can be simpler if only one of such methods is in use). The high-frequency current generated by the external unit (9) with a signal generator is modulated with a modulator (22). Note that in Figure 5 the switching elements (15) (see Figure 3) for managing multiple external electrodes (10, 10') are not displayed. The external unit (9) is further connected to corresponding interface and communications means, so as to exchange information to a third-party device, for instance, a storage device. A current sensor (23) is connected to one of the external electrodes (10) and the demodulator (12').

A particular embodiment of the capsule (3) of the implant (1) is shown in Figure 6A, wherein the hermetic capsule (3) consists of a thin and elongated configuration comprising dielectric segments (3') interspersed with conductive segments (3"), wherein the electronic circuit (5) of the implant (1) is fully contained within the capsule (3). In this particular embodiment, the implant (1) can consist only of the capsule (3), wherein the conductive segments act as or are connected to the electrodes (4, 4', 11, 11',11") (shown in Figures 1-2) of the implant (1). This implant (1) configuration is suitable for providing intraparenchymal brain stimulation or to record brain activity. The capsule (3) is elongated. In this case, the capsule (3) exhibits a circular cross-section, but other elongated shapes are also equally advantageous for the capsule (3), for instance, with elliptic, square, rectangular, or non-uniform cross-sections.

In the embodiment of the invention included in Figure 6A, two of its conductive segments (3"), preferentially at opposite ends, will be connected to the volume conduction electrode pair (4, 4') (see in Figures 2-3) for picking up a portion of the high-frequency (HF) current flowing across tissues by volume conduction and, optionally, for performing other tasks such as communications, stimulation and biopotentials recording. The additional conductive segments (3") can be also connected to recording and/or stimulation electrodes (11, 11') (see in Figures 2-3) or as structural parts. The conductive segments (3") are preferably made of a biocompatible metal (e.g., platinum, gold, stainless steel, and titanium) or alloy (e.g., platinum-iridium 90:10) and are optionally, completely, or partially coated with a material to improve their electrical performance (e.g., titanium nitride) or to improve their biocompatibility or other aspects such as lubricity (e.g., parylene). The internal cavity of the capsule (3), where the electronic circuit (5) is housed, is hermetic to protect the electronics. In this regard, the dielectric segments (3') are preferably made of glass or alumina that can be engineered to form a hermetic sealing with the material of the conductive segment (3"), either directly or using a filler material (brazing).

An alternative embodiment of the implant (1) is displayed in Figure 6B, wherein the conductive segments (3") of the capsule (1) are wired to the implant electrodes (4, 4') (see in Figures 2-3) with wires (24). The wires (24) may be covered with an insulation to insulate said wire leads (24) from each other. The set comprising the capsule (3) and the wires (24) is covered by a non-hermetic insulation (25). The non-hermetic insulation (25) may comprise one of the following: a sheath or tubbing of dielectric material (e.g., a biocompatible material such as silicone, polytetrafluoroethylene, polyurethane), a coating of dielectric material (e.g., a biocompatible material applicable as a coating such as silicone or parylene) or a rigid encapsulation of dielectric material (preferably biocompatible epoxy).

The wires (24) can be insulated from each other by setting a sufficient physical separation in a dielectric matrix. The wire leads (24) may comprise conductive tracks on a dielectric substrate. The wire leads (24) can be electrically and mechanically connected to the conductive segments (4, 4', 3") by any known methods in the field (e.g., spot welding or laser welding).

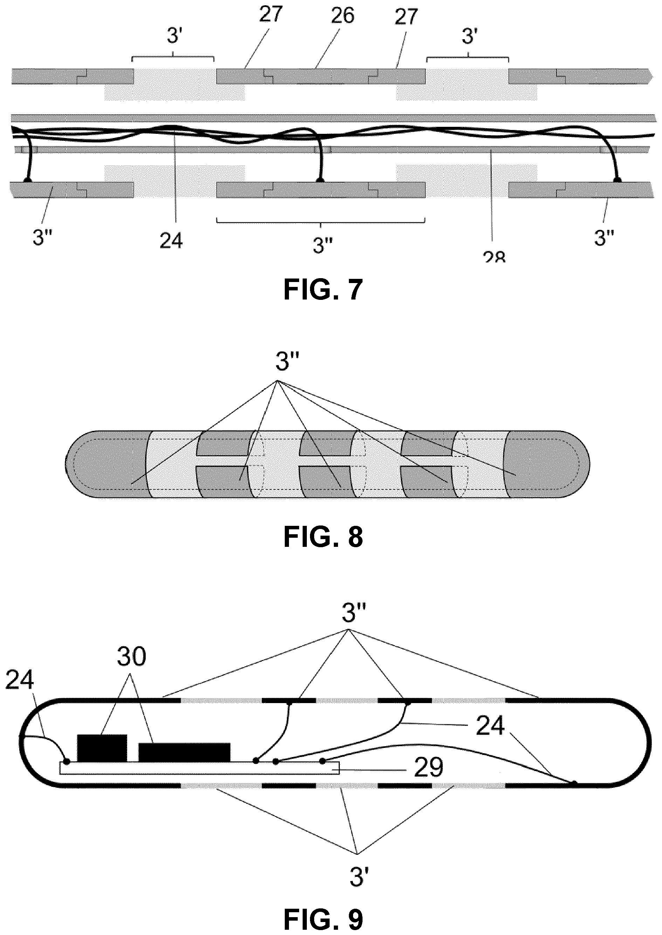

A detailed embodiment for implementing the hermetic capsule (3) of the device according to the invention is included in Figure 7, which comprises dielectric segments (3') interspersed with conductive segments (3"). In this Figure, the electronic circuit (5) has been omitted, for the sake of simplicity. The dielectric segments (3') comprise crystal, glass or ceramic cylinders. Each of the conductive segments (3") comprises a metal ring (26) hermetically sealed to the dielectric segments (3') along with a metal cylinder (27) welded to the metal ring (26). Moreover, within the hermetic capsule (3) there are a plurality of wires (24) connecting the conductive segments (3") to the electronic circuit (5) (not shown in Figure 7). In even more preferred embodiments of the hermetic capsule (3), it further comprises an internal tubbing (28) for improving mechanical robustness and, optionally, for storing the plurality of wires (24).

Figure 8 illustrates the possibility of implementing conductive segments (3") that do not encircle the entire cross-section of the capsule (3). This can be done in order to maximize the number of hermetic connections (19) to electrodes or, if these segments are directly used as the electrodes (4, 4',11, 11', 11", 11''') (Figures 2-3) of the implant (1), in order to maximize spatial and resolution selectivity for stimulation or for recording.

Figure 9 shows a possible internal implementation of the capsule (3) in which the arrangement of the electronic circuit (5) has been detailed. A plurality of wires (24) is used to connect the electronic circuit (5) with the conductive segments (3"). The electronic circuit (5) comprises a substrate (29) (e.g., a printed circuit board, PCB) on which the electronic components (30) are mounted.

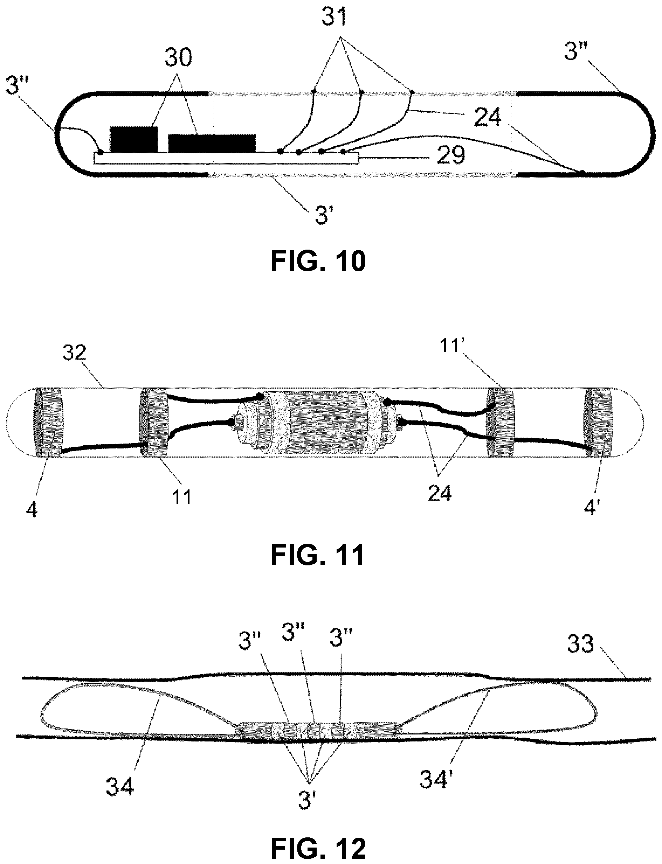

Instead of using multiple dielectric segments (3') which are engineered to form hermetic sealings with multiple conductive parts, the dielectric segment (3') can be implemented forming a hermetic continuous ring as in Figure 10, with conductive elements (31) embedded in the dielectric segment (3') such as metallic protrusions, pins or wires. This capsule (3) can be manufactured with known methods such as those used in High Temperature Co-fired Ceramics (HTCC) or Low Temperature Co-fired Ceramics (LTCC) in which metallic elements and dielectric substrates are fired in a kiln at the same time.

The conductive elements (3", 31) of the capsule (3) can be electrically connected to the electronic circuit (5) using known methods such as soldering, welding, wire bonding and flip chip bonding, among others.

An alternative embodiment of the implant (1) is displayed in Figure 11. Figure 11 provides two possible additional configurations (one for the capsule (3) and another one for the implant (1) as a whole):

a) The capsule (3), instead of forming a body with a uniform section, comprises stepped segments in which dielectric segments (3') are interspersed with conductive segments (3"). This configuration allows connecting wires (24) to the conductive segments (3") without increasing the overall diameter of the arrangement.

b) The implant (1) is preferably shaped as a semi-flexible threadlike device wherein the hermetic capsule (3) is contained within a non-hermetic flexible body (32) made of insulating material that also contains the wires (24) to connect the capsule (3) to the electrodes (4, 4', 11, 11'). The non-hermetic flexible body (32) can consist in a solid or a sheath or tubbing of dielectric material (preferably a biocompatible material such as silicone, polytetrafluoroethylene, polyurethane).

The wires (24) in Figure 11 can be insulated with a sheath or tubbing of dielectric material (preferably a biocompatible material such as silicone, polytetrafluoroethylene, polyurethane) or a coating of dielectric material (preferably a biocompatible material applicable in the form of coating such as silicone or parylene). Alternatively, the wires (24) can be insulated between them by physical separation in a dielectric matrix. For instance, the wires (24) can consist of conductive tracks on a dielectric substrate. These wires can be electrically and mechanically connected to the conductive segments (3") and to the electrodes (4, 4', 11, 11') by known methods (e.g., they can be welded to the conductive segments (3") by means of spot welding or laser welding). The conductive segments (3") and the electrodes (4, 4', 11, 11') are made of a biocompatible metal (e.g., platinum, gold, stainless steel, and titanium) or alloy (e.g., platinum-iridium 90:10) and are optionally, completely, or partially coated with a material to improve their electrical performance (e.g., titanium nitride) or to improve their biocompatibility or other aspects such as lubricity (e.g., parylene).

Figure 12 illustrates an alternative embodiment of the implant (1) that can be easily deployed and secured within a blood vessel (33), such as an artery or a vein. The implant (1) comprises a rigid capsule (3) and two primary electrodes with the form of fixation elements (34, 34'). Said fixation elements (34, 34') secure the capsule (3) against the vessel (33) wall so that its electrodes are in contact, or very close, to it. This enables the implant (1) to stimulate adjacent excitable tissues or to detect biopotentials originating in neighbouring tissues. In Figure 12 the fixation elements (34, 34') are displayed as simple loops, but they can also consist in other curved shapes adapted to secure the capsule (3) within the vessel (33) and, ideally, against its wall. These fixation elements (34, 34') are preferably made of a superelastic material (e.g., nitinol) so that the implant (1) can be easily squeezed in a minimally invasive device (e.g., catheter) and deployed in the vessel (33) by expelling from the minimally invasive device.

The fixation elements (34, 34') can be made of a conductive or non-conductive material. If conductive, these fixation elements (34) can constitute, totally or partially, electrodes (4, 4",11, 11', 11") of the implant (1). Preferably, as illustrated in Figure 11, these fixation elements (34, 34') are totally or partially conductive and are located at the opposite ends of the implant (1) axis and are part of the volume conduction electrode pair (4, 4') (Figures 1-3). This configuration maximizes the contact area of the volume conduction electrode pair (4, 4') and the distance between them, thus maximizing the power that can be collected through them. In other to further increase the distance between the electrodes of the volume conduction electrode pair (4, 4'), the fixation elements can be partially insulated so that only their distal portions are exposed.

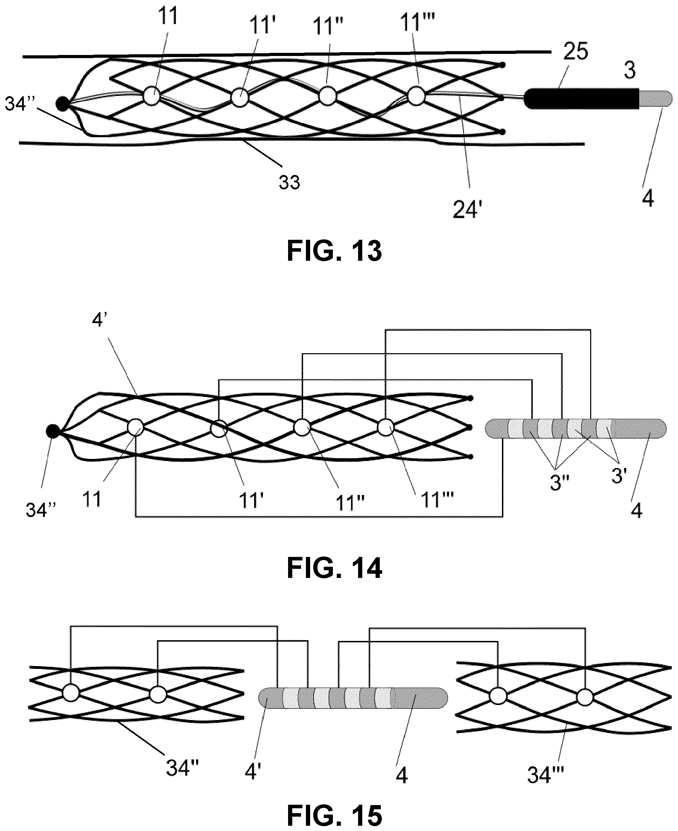

Figure 13 illustrates a further alternative embodiment of the implant (1) that can also be easily deployed and secured within a blood vessel (33), such as an artery or a vein. In this case the fixation elements (34") consist of hollow cylindrical meshes of the sort known as stents and that are used in vascular angioplastic procedures. Several electrodes (11, 11', 11", 11‴) are mounted on or integrated in the mesh so that they are in contact with the vessel (33) wall. A plurality of electrical connections is stablished between the electrodes (4', 11, 11', 11", 11‴) in or on the stent-like structure (34") and the capsule (3). These electrical connections can be implemented as wires (24) in a multiwire cable (24'). In Figure 13, the exposed conductor of the capsule (3) acts as one of the electrodes (4) of the volume conduction electrode pair (4, 34"). Note that neither the electrodes of the volume conduction electrode pair (4, 4') nor the capsule (3) have to be necessarily in contact with the vessel (33) wall. The mesh can be made of a dielectric material (e.g., polymer) or of a conductive material (e.g., nitinol). If the mesh is made of a conductive material, the recording and/or stimulation electrodes (11, 11', 11", 11‴) can be electrically insulated from it with intermediate dielectric layers. The electrodes can be glued to the mesh, for instance using an epoxy. Alternatively, the mesh can be implemented so that it monolithically integrates the electrodes (4', 11, 11', 11", 11‴) and the electrical connections which are electrically insulated in a dielectric substrate. Figure 14 shows the electrical connections for the configuration of Figure 13 wherein the insulators are not shown, for the sake of clarity.

Figure 15 illustrates in a preferred embodiment of the invention the possibility of using two or more fixing structures (34", 34‴) such as two stent-like segments to form part of the implant (1). This configuration is useful for developing implants (1) that can be deployed within blood vessels (33) that are tortuous or that branch into narrower vessels (33).

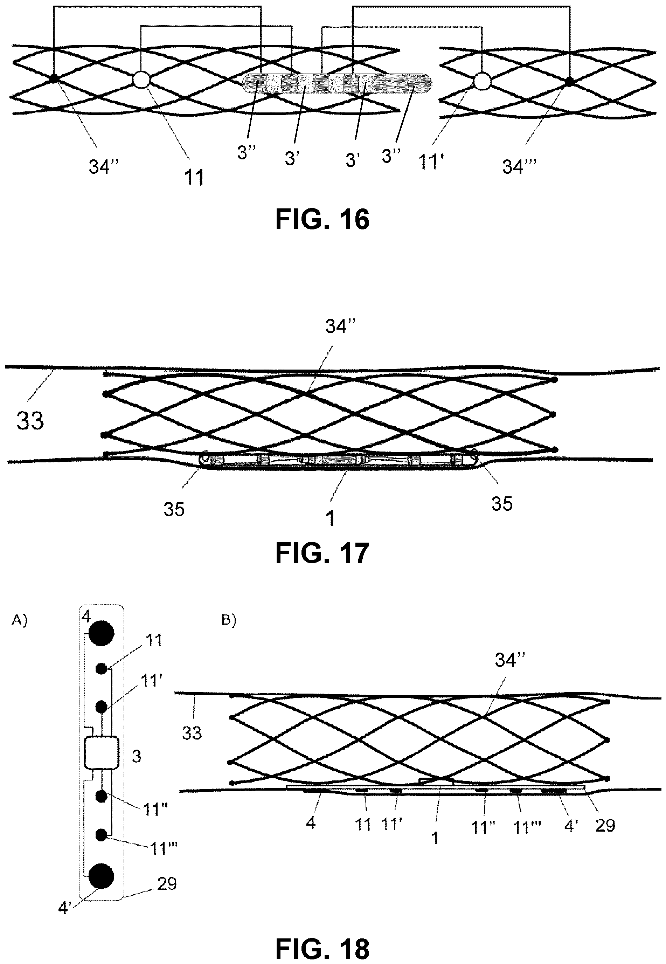

Figure 16 illustrates an embodiment of the implant (1) with stent-like structures (34", 34‴) as electrodes of the implant (1), wherein the connection between the stents (34", 34‴) and the electrical elements (3") comprise stimulation and/or recording electrodes (11, 11'). Preferably, as illustrated in the figure, these stent-like structures (34", 34‴) are conductive and are located at the opposite ends of the implant (1), being also part of the volume conduction electrode pair (4, 4') (Figures 1-3) of the implant (1). This maximizes the area of the volume conduction electrode pair (4, 4') and their distance, thus maximizing the collected power through them.

As shown in Figure 17, in another configuration of the invention, a stent-like structure (34") can be used to secure the position of the whole implant (1) (under any of its possible embodiments) against the vessel wall. This configuration is advantageous since it avoids mounting and wiring electrodes on or around the stent-like fixing structure (34"). In contrast to certain previous figures in which the capsule (3) is displayed in the middle of the vessel (33) lumen, Figure 17 also illustrates the possibility of securing the capsule (3) in-between the stent-like fixing structure (34") and vessel (33) wall. This configuration minimizes impact on blood flow and promotes absorption of the implant (1) by the vessel (33). The electrical parts of the implant (1) can be fastened with one or more fasteners (35) to the stent-like structure (34") thus forming a single device. It is worth underlining that in Figure 17 a single set of electrical parts of the implant (1) is shown.

Figure 18 illustrates the possibility of developing implants (1) than can be used in combination with stent-like structures but in which the stent-like structure (34") is not part of the implant (1) itself. As in the case of the previous configuration of Figure 17, this configuration avoids mounting and wiring electrodes on or around the stent-like structure, thus enabling easier manufacturing techniques. An advantage with respect the previous configuration of Figure 17 is allowing the clinician to choose among different stent-like structures (34") (or combinations thereof) to better match the geometrical and mechanical properties of the vessel (33). Figure 18 corresponds to a planar implant (1) configuration that can be manufactured by photolithographic processes. The dielectric substrate (29) is preferably made of a flexible or conformable and biocompatible material such as silicone or flexible or conformable thermoplastics such as polyethylene terephthalate. Figure 18A corresponds to a top view of the implant (1), while Figure 18B corresponds to a lateral view of the implant together with the stent-like fixing structure (34") used to secure it to the blood vessel (33).

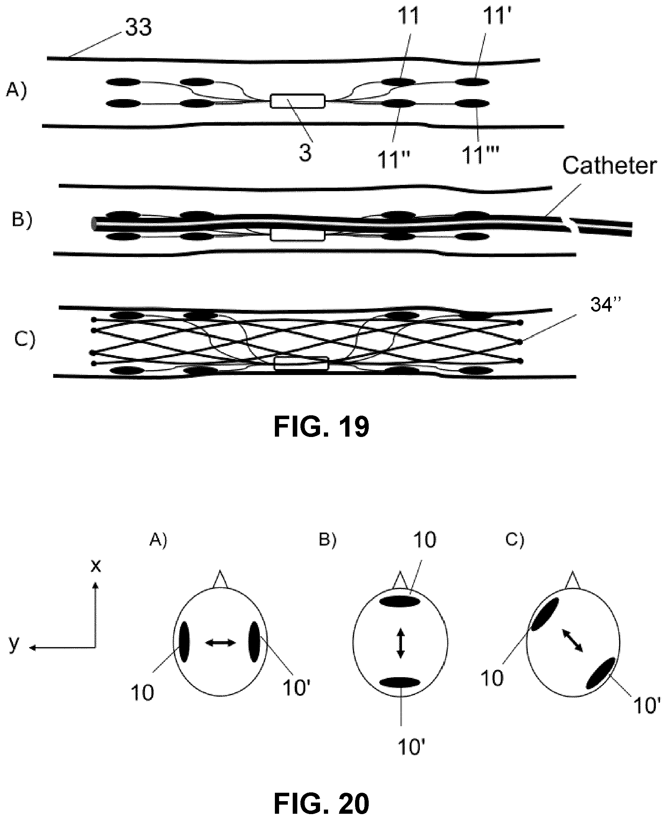

Figure 19 also illustrates the combination of an endovascular implant (1) and a stent-like fixing structure (34") but in which the stent-like fixing structure (34") is not part of the implant (1) itself. In this case the endovascular implant (1) consists of an elongated non-rigid and collapsed structure which expanded and secured to the vessel by means of the stent-like structure (34"). Such non-rigid and collapsed structure is created by forming a hollow network of connections between the electrodes (11, 11', 11", 11‴) and the capsule (3) by means of wires (24) which are flexible or plastically deformable. To deploy this implant (1), three steps are followed: A) Positioning the collapsed implant (1) within the vessel. B) Introducing the stent-like structure within the vessel (33) by using a catheter. C) Deployment of stent-like structure (34") to cause implant (1) expansion and fixation to the vessel (33).

The strategy to design external electrode (10, 10') arrangements across which the current is injected for the implants (1) can be stated as: coarsely place the two external electrodes (10, 10') at the two projected points on the head or neck surface of the trajectory defined by the implant predominant orientation (implant axis). Figures 20A-C illustrate two-electrode arrangements for powering and communicating with implants according to the invention which are predominantly oriented in a single direction. Particularly, three implant (1) orientations are shown (marked in the drawing as a double-headed arrow): A) From one ear to another (along y-axis), B) From the forehead to the nape (along x-axis), C) Diagonal.

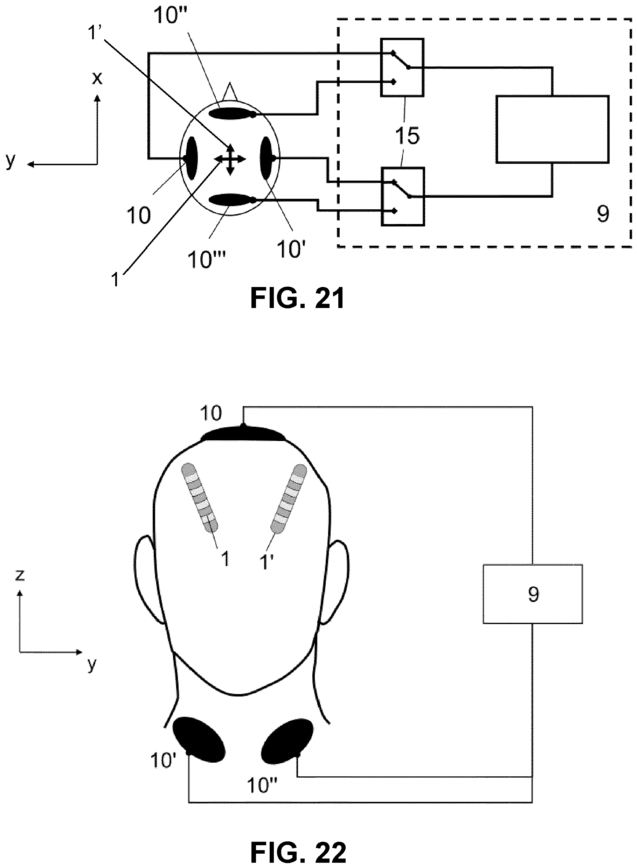

In a preferred embodiment of the invention, for targeting multiple body regions, it comprises multiple implants (1) that may be oriented in different directions. For illustration, in Figure 21, two implants with predominant orientations perpendicular to each other are arranged. In cases like this, that is, in case of multiple implants exhibiting substantially different predominant orientations, it is possible to powering and communicating with them by using multiple external electrode pairs (10, 10', 10", 10''') and an external unit (9) with switching elements. In Figure 21, the first implant (1) is powered and communicated with via a first pair of the external electrodes (10, 10') and the second implant (1') is powered and communicated with via a second pair of external electrodes (10", 10'''). Said external unit (9) comprises one or more switching elements (15) for selective activation of one electrode pair (10, 10') or another (10", 10'''). The switching elements (15) comprise, for instance, relays and/or analogic multiplexers (single-channel or multi-channel). The use of switching elements (15) in the external unit (9) enables to select each time a particular external electrode pair, and to power and communicate with the implants that are predominantly oriented in the direction defined by the external electrode pair.

Figure 22 illustrates a preferred embodiment of the invention in which one or more implants, not displayed at scale, are oriented predominantly in the vertical direction (aligned with the z axis according to the reference system depicted in Figure 22). In this embodiment, the arrangement of the external electrodes consists of two bottom external electrodes (10', 10") and a top external electrode (10). Said arrangement, in addition to enabling both powering and communication by means of volume conduction with implants (1, 1') that are oriented predominantly in the vertical direction, enables a more cosmetically appealing setup for the user that wears the external electrodes: the top external electrode (10) can be hidden beneath a wig, while the bottom electrodes around the neck region can be hidden behind the collar of a shirt. In other embodiments of the invention, there are at least two top electrodes and/or at least two bottom external electrodes (10', 10") on the neck region, since they are advantageous for symmetry in the design and to minimize the contact impedance, as well as to provide an easier monitoring of such contact impedance. However, the invention would still be operative with a single bottom or top electrode.

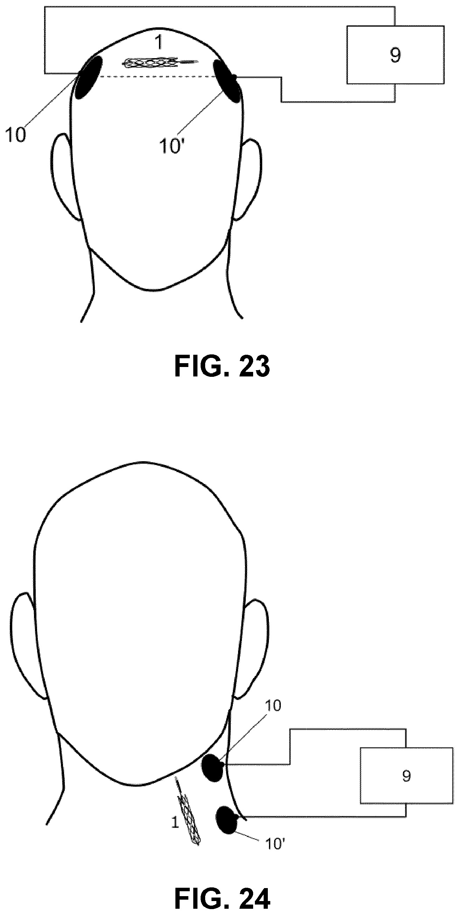

Other preferred embodiment comprising a leadless endovascular implant, not displayed at scale, with two or more external electrodes (10, 10') is displayed in Figure 23. This arrangement is suitable for powering and enabling communications with the neurovascular stent by means of volume conduction with high-frequency currents. The external electrodes are connected to an external unit (9). As indicated above, in some embodiments, the implant (1) is connected to a neurovascular stent within a blood vessel with a short multiwire cable or a plurality of wires with a length lower than 5 cm.

Figure 24 illustrates a possible external electrode (10, 10') arrangement for powering and communicating with, by means of volume conduction at high frequency, a leadless endovascular implant according to the invention which is implanted within the jugular internal vein or the jugular internal artery to perform stimulation of the vagus nerve. The external unit (9) and the external electrodes (10, 10') can be integrated in a single handled unit that the patient can manually position on the neck when stimulation is required. The implant (1), not displayed at scale, is aligned with the external electrodes (10, 10').

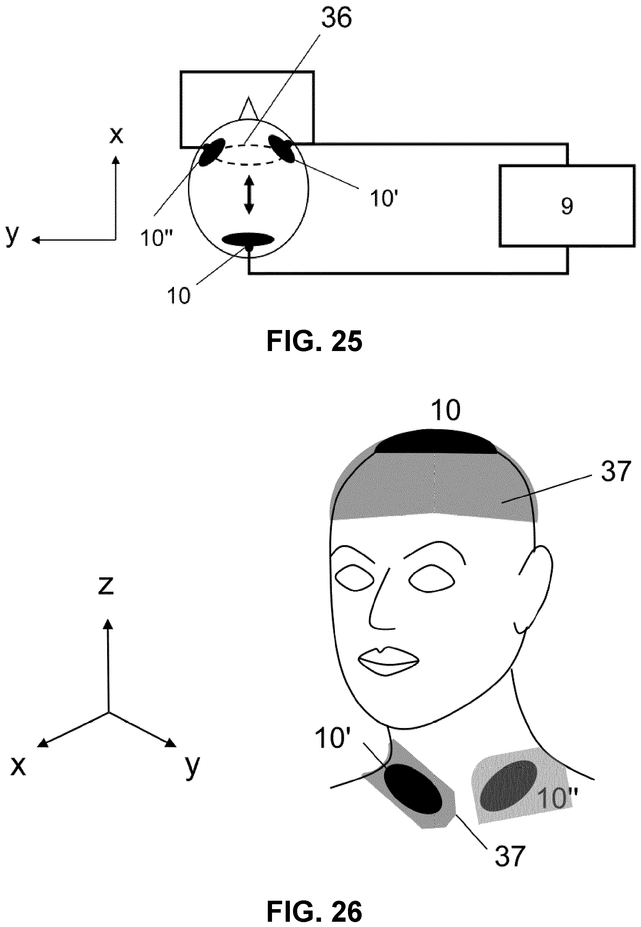

In cases in which it is either not possible or not practical to place an external electrode at one of the projected points on the head surface of the trajectory defined by the implant (1) longitudinal axis, it is possible to create a so-called virtual electrode by short circuiting at least some of the external electrodes (10, 10', 10"). This arrangement of electrodes brings more freedom in the design of the most appropriate location so that they affect minimally the daily life of the wearer of the system. An embodiment of this virtual electrode (36) is shown in Figure 25, wherein the projected point defined by the trajectory of the implant (1) is between the eyes. To reach this projected point, the device comprises three external electrodes (10, 10', 10") arranged in a triangular configuration. The external unit (9) connects with the external electrodes (10, 10', 10").

In certain embodiments of the invention, the external electrodes (10, 10', 10") are part of an electrically passive securing structure (37), for example, one of the following: headset, headband, helmet, cap, bandana, or scarf. In Figure 26, the external electrodes (10, 10', 10") are fixed with headbands which eases donning and doffing them.

The external electrodes (10, 10') can be implemented in diverse ways, for instance, as dry electrodes which do not require any fluid of gel to improve their electrical coupling with the skin. Higher frequencies for power transfer and communications ease the use of such dry electrodes because the electrical conduction across the contact area between the external electrodes (10, 10') and the tissues is mainly capacitive and not galvanic. In fact, it is possible to employ electrodes with a thin dielectric layer on its surface. These electrodes can be manufactured with materials well tolerated by the skin like the stainless steel or, for greater comfort and conformability, with conductive fabrics or conductive polymers (e. g, carbon filled rubber). To facilitate electrical contact across hair, the external electrodes can contain protrusions and/or indentations, for instance, shaped as bristles or blunt spikes.

The external electrodes (10, 10') are arranged to ensure that thin implants (1) within the head or neck are able to pick-up powers in the order of a few mW or tenths of mW when applying external ac currents through the external electrodes (10, 10') that comply with electrical safety standards. Preferably, for minimal invasiveness, the implants (1) exhibit a diameter below 2 mm and lengths in the order of a few millimetres (3-10 mm) or few centimetres (1-5 cm).

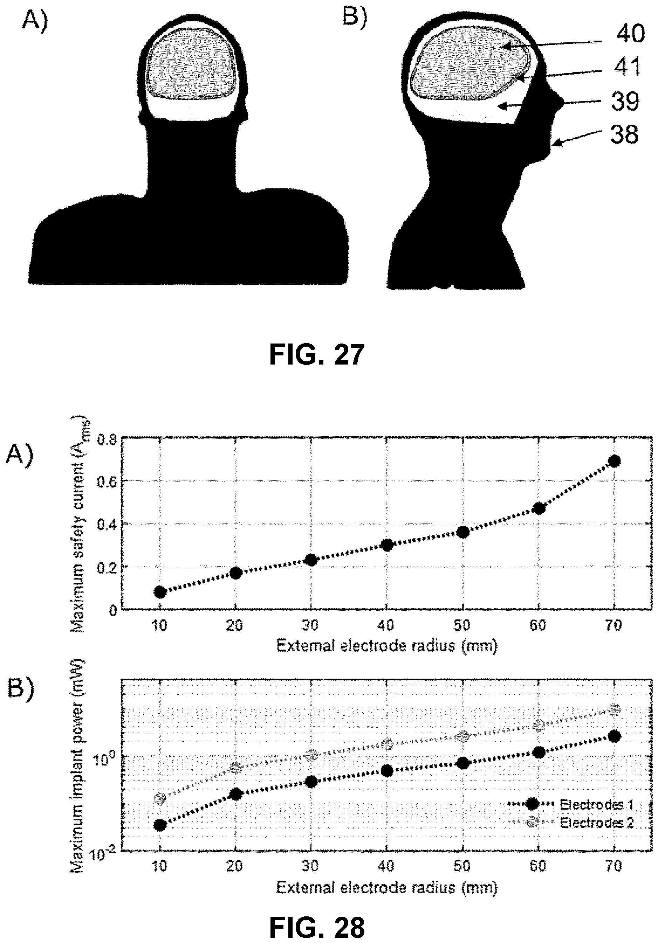

For validating the advantages of the present invention, numerical simulations (see Figures 27-34) have been carried out in order to determine the general geometrical requirements (e.g., size and location) and arrangements for the external electrodes (10, 10'). The external electrodes must maximize the power delivered to the implants (1) while ensuring that the spatial averaged specific absorption rate (SAR, corresponding to the power absorbed per mass of tissue) is below 10 W/kg as imposed by safety standards.

First, the maximum power that implants (1) can harvest has been numerically calculated. The implants (1) are placed several centimetres inside the human brain parenchyma. The simplified anatomical model consisted of the head, the neck, and the upper part of the torso of a young male adult (see Figure 27), according to Duke's model from Virtual Family [M. Gosselin et al., "Development of a new generation of high-resolution anatomical models for medical device evaluation: the Virtual Population 3.0.," Physics in medicine and biology, vol. 59 18, pp. 5287-303, 2014]. The tissue composition has been simplified, only considering four tissues: muscle (38), skull bone (39), brain white matter (40) and brain grey matter (41). These tissues are modelled considering their conductivity, permittivity (for a frequency of 6.78 MHz) and density, as summarized in Table 1.

Table 1. Electrical parameters and mass density of the modeled tissues.

| Tissue | Conductivity (S/m) at 6.78 MHz | Relative permittivity at 6.78 MHz | Density (kg/m$^3$) |
|---|---|---|---|
| Muscle | 0.6 | 233 | 1090 |
| Skull | 0.12 | 90 | 1178 |
| Grey matter | 0.25 | 397 | 1045 |
| White matter | 0.14 | 206 | 1041 |

The analysis was performed at 6.78 MHz because this is a preferred fundamental frequency for the applied high-frequency currents as it avoids the risk of neuromuscular stimulation, as well as this frequency corresponds to the central frequency of the first ISM (Industrial, Scientific and Medical) band after 5 MHz. We recall that the safety standards consider that frequencies above 5 MHz can hardly produce neuromuscular stimulation (see for instance ["IEEE Standard for Safety Levels with Respect to Human Exposure to Radio Frequency Electromagnetic Fields, 3 kHz to 300 GHz", 2019.]) The use of much higher frequencies (>100 MHz) is not convenient because, due to the skin effect, the electric field induced in the middle regions of the body would be substantially lower.

The simulations have been carried out using a simulation platform (COMSOL Multiphysics 5.5 from COMSOL Inc.) based on the finite element method. The geometry of the implants (1) has been simplified as two conductive cylindrical electrodes separated a few centimetres apart. Two implant geometries are considered. The dimensions of the electrodes of the volume electrode pair (4, 4') for the first case are: a diameter (D) of 1 mm, an electrode length (L) of 2 mm and a separation (S) between electrodes of 30 mm; and for the second case: D = 2 mm, L = 4 mm, and S = 40 mm. The implant electrodes (4, 4', 11) are placed inside white matter (40) at more than 10 mm of separation from the grey matter (41). The simulated Thévenin impedance ($Z_{Th}$) between both implant electrodes (4, 4', 11) is 1060-590i $\Omega$ for the first case, and 527-270i $\Omega$ for the second one.

The externally delivered voltage or current produces an electric field within the tissues and a small portion of the energy associated to this electric field can be picked up by the implants (1). As described in [M. Tudela-Pi et al., "Powering implants by galvanic coupling: A validated analytical model predicts powers above 1 mW in injectable im-

$$P_{im} = \frac{V_{Th}^2}{4Z_{Th}}$$

plants,"IFMBE Proceedings, vol. 68, no. 3, 2019], the maximum power ($P_{im}$) collected by an implant is:

, where $V_{Th}$ is the open circuit voltage that the implant (1) receives and the impedance $Z_{Th}$ can be considered purely resistive.

Relatively large external electrodes (10, 10'), with a total area larger than 1 cm$^2$, are required to obtain powers above hundreds of $\mu$W in the implants (1). Otherwise, the SAR limitation is exceeded in the vicinity of external electrodes (10, 10') as excessive current densities are produced for delivering a current magnitude capable of creating an adequate field magnitude at the location of the implants (1). By increasing the external electrode (10, 10') area, it is possible to deliver higher currents (or voltages) thereby creating higher fields at the location of the implants. However, the electrode size will be physically limited by anatomical constraints. Furthermore, it is neither necessary nor convenient to deliver very high currents. Figure 28A shows that currents above 0.8 A$_{RMS}$ can induce tissue overheating in the neck region since the neck section is smaller than the head one, so the current density and the electric field can exceed the safety limits. For these reasons, external electrodes (10, 10') with areas over 250 cm$^2$ are not advantageous over smaller ones.

It has been found that it is beneficial to coarsely place the external electrodes (10, 10') at the body surfaces that intersect with the vector projection defined by the implant longitudinal axis. This configuration not only allows focusing the field but also tends to produce an electric field parallel to the implant electrodes (10, 10') which also maximizes the collected power. The results also indicate that by using arrangements with multiple electrodes (10, 10'), it is possible to deliver higher currents than with a single pair of external electrodes (10, 10') (even if the total area of the electrodes (10, 10') is the same).

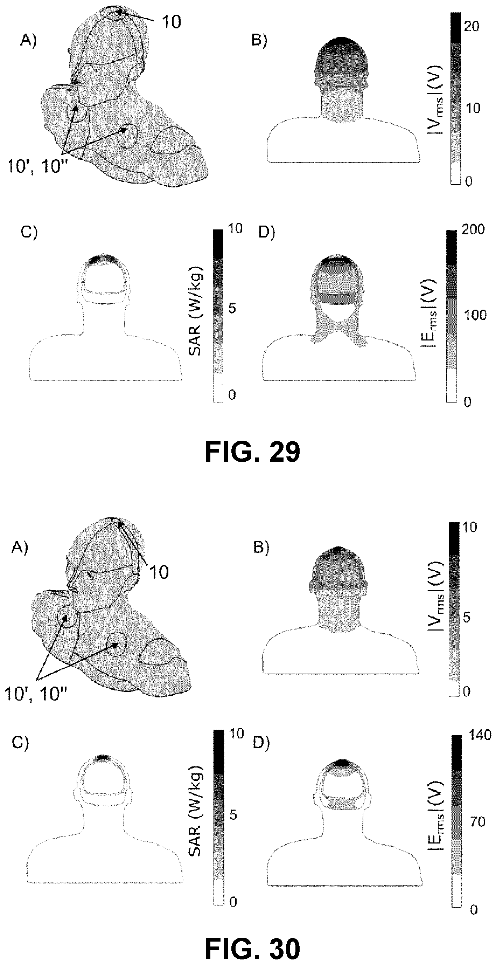

Some external electrodes (10, 10') arrangements and locations allow focusing the electric field where the implants (1) are located, thereby maximizing the collected power. The results of a three-electrode arrangement shown in Figure 28B (and Figure 29) demonstrate that thin and elongated implants (1) reach powers from hundreds of $\mu$W to several mW. Two implant configurations have been shown: "Electrodes 1": D (diameter) = 1 mm, L (length)= 2 mm, S (separation) = 30 mm; and "Electrodes 2": D = 2 mm, L = 4 mm, S = 40 mm.

Figure 29A shows the geometry of a three-electrode (10, 10', 10") vertical arrangement, with two interconnected external electrodes (10', 10") placed around the neck and a single external electrode (10) placed at the top of the head, with a radius of 3 cm. The delivered current has been 0.29 A$_{rms}$. Figure 29B corresponds to the voltage distribution in the frontal plane, wherein the local SAR is maximum. Figure 29C illustrates the SAR distribution while Figure 29D shows the electric field distribution, in the same plane as Figure 29B. Note that in Figures 29-32, it has been displayed the averaged SAR distribution (over 10 grams cube of the head or neck body region) and voltage distribution for different electrodes size.

Figure 30A shows the geometry of a three-electrode vertical arrangement, with two interconnected electrodes placed around the neck and a single electrode placed at the top of the head, with a radius of 3 cm. The delivered current has been 0.08 A$_{rms}$. Figure 30B corresponds to the voltage distribution in the frontal plane, wherein the local SAR is maximum. Figure 30C illustrates the SAR distribution while Figure 30D shows the electric field distribution, in the same plane as Figure 30B. From the previous results of Figures 29-30 one observes that, if at least one electrode is placed on the top of the head area and at least one electrode is placed around the neck, an electric field with a main vertical component appears within the tissues of interest when an external current is delivered.

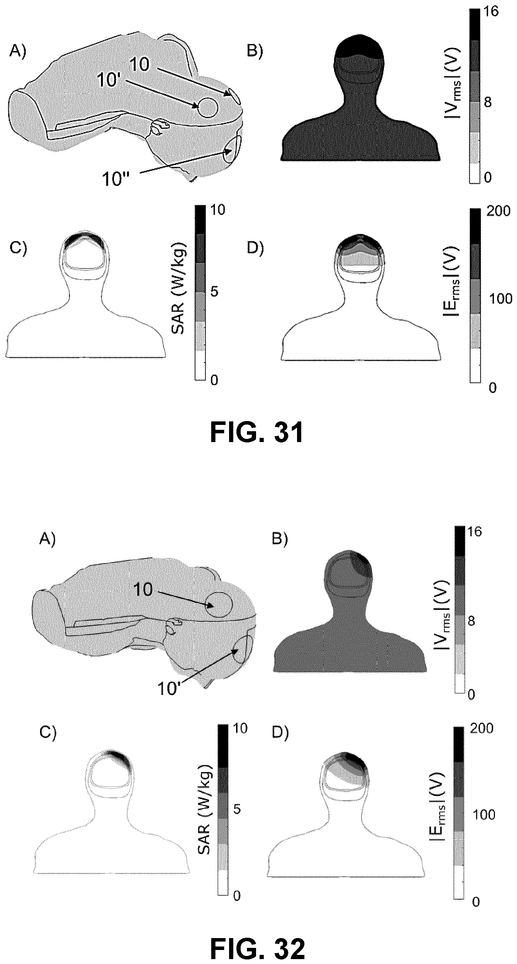

On the other hand, if the electrodes are horizontally positioned, the electric field has a main horizontal component. Figure 31A shows the geometry of a three-electrode horizontal arrangement, with two interconnected external electrodes (10, 10') placed at the top of the parietal region and a single external electrode (10") placed at the top of the frontal region, wherein the parietal electrodes (10, 10') have a radius of 2 cm, and the front electrode (10") has a radius of 3 cm. The delivered current has been 0.34 A$_{rms}$. Figure 31B corresponds to the voltage distribution in the frontal plane, wherein the local SAR is maximum. Figure 31C illustrates the SAR distribution and Figure 31D shows the electric field distribution.

Figure 32A shows the geometry with two external electrodes (10, 10'), comprising a single top external electrode (10) placed at the top of the parietal region, and a single external electrode (10') placed at the top of the frontal region, with a radius of 2.82 cm (parietal electrode) and 3 cm (frontal electrode), respectively. The delivered current has been 0.26 A$_{rms}$. Figure 32B corresponds to the voltage distribution in the frontal plane, wherein the local SAR is maximum. Figure 32C illustrates the SAR distribution and Figure 32D shows the electric field distribution, in the same plane as Figure 32B.

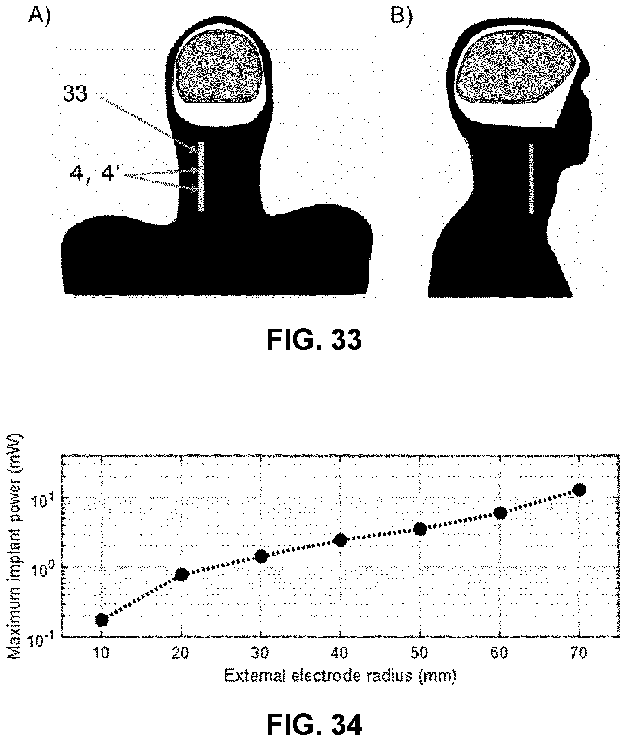

In a second set of simulations, the implants (1) are deployed within blood vessels (33) of the head and neck. Figure 33(A-B) corresponds to a scenario in which a single implant (1) is deployed within the internal jugular vein for performing stimulation of the adjacent vagus nerve. A blood cylinder (length of 100 mm, and radius equal to 5 mm), mimicking the internal jugular, has been included. Within this cylinder the volume conduction electrode pair (4, 4') is placed (D = 1 mm, L = 2 mm, S = 30 mm, as illustrated in Figure 33). The separation between the external part of the electrodes (4, 4') and the cylinder wall is 0.5 mm. The electrical properties of blood tissue at 6.78 MHz are: conductivity equal to 1.06 S/m, and relative permittivity of 421. The tissue density was approximated to 1000 kg/m$^3$. The impedance between the volume conduction electrode pair (4, 4') was 212-33i $\Omega$.

Finally, Figure 34 illustrates that powers of several mW can be collected by an implant (1) within the jugular vein without exceeding a safety threshold of local SAR above 10 W/Kg.

**Claims**

1. System for powering or controlling the electronics of head or neck implants by volume conduction, comprising:

> - at least one elongated implant (1), adapted to be implanted inside a body region (2), said body region (2) being comprised in a human or animal head or neck, wherein the implant (1) comprises:

a) a hermetic capsule (3);

b) a plurality of primary electrodes, comprising a volume conduction electrode pair (4, 4'); said primary electrodes being arranged, at least in part, externally to the capsule (3);

c) an electronic circuit (5) housed inside the capsule (3) and electrically connected to the primary electrodes, comprising:

- a digital control unit (6);

- a power transfer stage (7) adapted to receive at least part of the energy of a high-frequency current transmitted by volume conduction through the volume conduction electrode pair (4, 4'), and to transfer the power associated to said at least part of the energy of the high-frequency current to the digital control unit (6) and to a communications stage (8);

- a communications stage (8) adapted to detect, across two of the primary electrodes, the high-frequency current transmitted by volume conduction to the volume conduction electrode pair (4, 4'); and to process the high-frequency current by means of the digital control unit (6); and

- an external unit (9) adapted to generate at least the high-frequency current to transfer power to and to communicate with the at least one implant (1); wherein the fundamental frequency of the high-frequency current is comprised between 1 MHz and 200 MHz; wherein

the hermetic capsule (3) of the implant (1) is elongated and comprises one or more dielectric segments (3') interspersed with one or more conductive segments (3"); and

the external unit (9) is electrically connected to a plurality of external electrodes (10, 10'), wherein at least two of said external electrodes (10, 10') comprise corresponding contact areas adapted to be arranged over the body region (2), at substantially opposite positions along a longitudinal axis defined by the elongated implant (1); and wherein the sum of contact areas of each said external electrodes (10, 10') is comprised between 1 $cm^2$ and 250 $cm^2$.

2. The system according to the preceding claim, wherein the primary electrodes further comprise a plurality of communication electrodes; wherein the communications stage (8) of the electronic circuit (5) of the implant (1) is further adapted to exchange, through two of the primary electrodes, one or more management signals with the external unit (9) by volume conduction.

3. The system according to any of the preceding claims, wherein the electronic circuit (5) of the implant (1) further comprises a biopotentials acquisition stage (13) comprising amplifiers, band-pass filters, analog-to-digital converters and switching elements (15), said biopotentials acquisition stage (13) being adapted to record one or more electrical signals from the body region through at least the volume conduction electrode pair (4, 4') and/or one or more recording and stimulation electrodes (11, 11'), being the electrical signals dependent on a voltage difference outside the capsule (3).

4. The system according to any of the preceding claims, wherein the electronic circuit (5) of the implant (1) further comprises a neurostimulation stage (14) adapted to generate and deliver electrical signals outside the implant (1) through the volume conduction electrode pair (4, 4') and/or the one or more recording and stimulation electrodes (11, 11'), said neurostimulation stage (14) comprising at least one of the following: a pulse generator (10), rectification elements (7') and switching elements (15).

5. The system according to any of the preceding claims, wherein one or more conductive segments (3") of the hermetic capsule (3) are electrically connected to one or more primary electrodes (4, 4") of the implant (1) or to one or more recording and/or stimulation electrodes (11, 11') of the implant (1), arranged externally of the capsule (3), by means of one or more wires (24); said implant (1) comprising a non-hermetic insulation (25) which houses, at least in part, the capsule (3) and the wires (24).

6. The system according to any of the preceding claims, wherein the hermetic capsule (3) further comprises an internal tubbing (28) to reinforce the structure and, optionally, to house the wires (24) which connect the conductive segments (3") to the electronic circuit (5).

7. The system according to any of the preceding claims, wherein the conductive segments (3") do not encircle the entire cross-section of the capsule (3).

8. The system according to any of the preceding claims, wherein the capsule (3) comprises stepped segments in which dielectric segments (3') are interspersed with conductive segments (3").

9. The system according to any of the preceding claims, wherein the implant (1) is shaped as a semi-flexible threadlike device and wherein the hermetic capsule (3) is contained within a non-hermetic flexible body (32) made of insulating material that also contains the wires (24) to connect the capsule (3) to one or more primary electrodes (4, 4") of the implant (1) or to one or more recording and/or stimulation electrodes (11, 11') of the implant (1).

10. The system according to any of the preceding claims, wherein the implant (1) comprises a stent-like structure (34") adapted to secure the position of the whole implant (1) against a vessel wall, and wherein the capsule (3) is externally fastened to the stent-like structure (34") by means of one or more fasteners (35).

11. The system according to any of claims 1-9, wherein the implant (1) is planar and comprises a dielectric substrate (29) made of a flexible or conformable material, and wherein the implant (1) is optionally arranged in contact with a stent-like structure (34") adapted to secure the position of the whole implant (1) against a vessel wall.

12. The system according to any of claims 1-9, wherein the implant (1) comprises an elongated non-rigid structure which can be expanded and secured to a vessel wall by means of the stent-like structure (34"), wherein the implant (1) further comprises a hollow network of connections between the recording and/or stimulation electrodes (11, 11') and the capsule (3) by means of wires (24) which are flexible or plastically deformable.

13. The system according to any of the preceding claims wherein:

   - the power transferring stage (7) of the electronic circuit (5) comprises a blocking capacitor (7''') connected in series with the volume conduction electrode pair (4, 4') or the plurality of communication electrodes, said blocking capacitor (7''') being adapted to prevent passage of dc current and to enable the flow of the high-frequency current to and from the electronic circuit (5);
   - the communications stage (8) comprises a demodulator unit (12) adapted to demodulate the high-frequency current; and
   - the digital control unit (6) is connected to a load modulator (17) adapted to modulate symmetrically or asymmetrically the load of the implant (1).

14. The system according to any of the preceding claims, wherein the external unit (9) comprises:

   - a power supply (21);
   - a digital control unit (6') and a modulator (22) adapted to generate high-frequency signals adapted to power and communicate with one or more implants (1) by volume conduction;
   - receiving means adapted to receive the one or more signals from the one or more implants (1) through the head or neck body region (2) by volume conduction;
   - processing means adapted to process information associated to the one or more signals received from the one or more implants (1); and
   - a signal amplifier (18') adapted to amplify the one or more management signals generated by the digital control unit (6') and the modulator (22); and to deliver said signals to one or more implants (1) through said head or neck body region (2) by volume conduction.

15. The system according to any of the preceding claims, wherein the external unit (9) comprises a plurality of external electrodes (10, 10', 10', 10"), and one or more switching elements (15) or permanent interconnections electrically connected to the one or more external electrodes (10, 10', 10", 10''') to selectively select a set of them for delivering the high-frequency current and/or to selectively short circuit one or more of them; and/or at least one external electrode (10, 10') adapted to be placed on the scalp; and at least one external electrode (10, 10') adapted to be placed at the neck region.

**Patentansprüche**

1. System zum Versorgen oder Steuern der Elektronik von Kopf- oder Halsimplantaten durch Volumenleitung, Folgendes umfassend:

   - mindestens ein langgestrecktes Implantat (1), das dazu angepasst ist, im Inneren einer Körperregion (2) implantiert zu werden, wobei die Körperregion (2) in einem menschlichen oder tierischen Kopf oder Hals umfasst ist, wobei das Implantat (1) Folgendes umfasst:

    a) eine hermetische Kapsel (3);
    b) eine Vielzahl von Primärelektroden, umfassend ein Volumenleitungselektrodenpaar (4, 4'); wobei die Primärelektroden mindestens teilweise extern zur Kapsel (3) angeordnet sind;
    c) eine elektronische Schaltung (5), die im Inneren der Kapsel (3) aufgenommen und elektrisch mit den Primärelektroden verbunden ist, Folgendes umfassend:

        - eine digitale Steuereinheit (6);
        - eine Leistungstransferstufe (7), die dazu angepasst ist, mindestens einen Teil der Energie eines Hochfrequenzstroms zu empfangen, der durch Volumenleitung durch das Volumenleitungselektrodenpaar (4, 4') übertragen wird, und die Leistung, die mit dem mindestens einen Teil der Energie des Hochfrequenzstroms assoziiert ist, an die digitale Steuereinheit (6) und an eine Kommunikationsstufe (8) zu transferieren;
        - eine Kommunikationsstufe (8), die dazu angepasst ist, über zwei der Primärelektroden den Hochfrequenzstrom zu detektieren, der durch Volumenleitung auf das Volumenleitungselektrodenpaar (4, 4') übertragen wird; und den Hochfrequenzstrom mittels der digitalen Steuereinheit (6) zu verarbeiten; und
        - eine externe Einheit (9), die dazu angepasst ist, mindestens den Hochfrequenzstrom zu generieren, um Leistung an das mindestens eine Implantat (1) zu transferieren und mit diesem zu kommunizieren; wobei die Grundfrequenz des Hochfrequenzstroms zwischen 1 MHz und 200 MHz umfasst;

    wobei

    die hermetische Kapsel (3) des Implantats (1) langgestreckt ist und ein oder mehrere dielektrische Segmente (3') umfasst, die mit einem oder mehreren leitfähigen Segmenten (3") durchsetzt sind; und
    die externe Einheit (9) elektrisch mit einer Vielzahl von externen Elektroden (10, 10') verbunden ist, wobei mindestens zwei der externen Elektroden (10, 10') entsprechende Kontaktflächen umfassen, die dazu angepasst sind, über der Körperregion (2) an im Wesentlichen entgegengesetzten Positionen entlang einer Längsachse, die durch das langgestreckte Implantat (1) definiert ist, angeordnet zu werden, und wobei die Summe der Kontaktflächen jeder der externen Elektroden (10, 10') zwischen 1 cm$^2$ und 250 cm$^2$ umfasst ist.

**2.** System nach dem vorhergehenden Anspruch, wobei die Primärelektroden ferner eine Vielzahl von Kommunikationselektroden umfassen; wobei die Kommunikationsstufe (8) der elektronischen Schaltung (5) des Implantats (1) ferner dazu angepasst ist, über zwei der Primärelektroden ein oder mehrere Verwaltungssignale mit der externen Einheit (9) durch Volumenleitung auszutauschen.

**3.** System nach einem der vorhergehenden Ansprüche, wobei die elektronische Schaltung (5) des Implantats (1) ferner eine Biopotentialerfassungsstufe (13) umfasst, umfassend Verstärker, Bandpassfilter, Analog-Digital-Wandler und Schaltelemente (15), wobei die Biopotentialerfassungsstufe (13) dazu angepasst ist, ein oder mehrere elektrische Signale aus der Körperregion durch mindestens das Volumenleitungselektrodenpaar (4, 4') und/oder eine oder mehrere Aufzeichnungs- und Stimulationselektroden (11, 11') aufzuzeichnen, wobei die elektrischen Signale von einer Spannungsdifferenz außerhalb der Kapsel (3) abhängig sind.

**4.** System nach einem der vorhergehenden Ansprüche, wobei die elektronische Schaltung (5) des Implantats (1) ferner eine Neurostimulationsstufe (14) umfasst, die dazu angepasst ist, elektrische Signale außerhalb des Implantats (1) durch das Volumenleitungselektrodenpaar (4, 4') und/oder die eine oder mehrere Aufzeichnungs- und Stimulationselektroden (11, 11') zu generieren und zuzuführen, wobei die Neurostimulationsstufe (14) mindestens eines von Folgendem umfasst: einen Impulsgenerator (10), Gleichrichtungselemente (7') und Schaltelemente (15).

**5.** System nach einem der vorhergehenden Ansprüche, wobei ein oder mehrere leitfähige Segmente (3") der hermetischen Kapsel (3) elektrisch mit einer oder mehreren Primärelektroden (4, 4") des Implantats (1) oder mit einer oder mehreren Aufzeichnungs- und/oder Stimulationselektroden (11, 11') des Implantats (1), die extern zur Kapsel (3) angeordnet sind, mittels eines oder mehrerer Drähte (24) verbunden sind; wobei das Implantat (1) eine nichthermetische Isolierung (25) umfasst, die mindestens teilweise die Kapsel (3) und die Drähte (24) aufnimmt.

**6.** System nach einem der vorhergehenden Ansprüche, wobei die hermetische Kapsel (3) ferner eine innere Verrohrung (28) umfasst, um die Struktur zu verstärken und gegebenenfalls die Drähte (24) aufzunehmen, die die leitfähigen Segmente (3") mit der elektronischen Schaltung (5) verbinden.

**7.** System nach einem der vorhergehenden Ansprüche, wobei die leitfähigen Segmente (3") nicht den gesamten Querschnitt der Kapsel (3) umschließen.

**8.** System nach einem der vorhergehenden Ansprüche, wobei die Kapsel (3) gestufte Segmente umfasst, in denen dielektrische Segmente (3') mit leitfähigen Segmenten (3") durchsetzt sind.

**9.** System nach einem der vorhergehenden Ansprüche, wobei das Implantat (1) als halbflexible fadenförmige Vorrichtung geformt ist und wobei die hermetische Kapsel (3) innerhalb eines nichthermetischen flexiblen Körpers (32) aus Isoliermaterial enthalten ist, der auch die Drähte (24) enthält, um die Kapsel (3) mit einer oder mehreren Primärelektroden (4, 4") des Implantats (1) oder mit einer oder mehreren Aufzeichnungs- und/oder Stimulationselektroden (11, 11') des Implantats (1) zu verbinden.

**10.** System nach einem der vorhergehenden Ansprüche, wobei das Implantat (1) eine stentartige Struktur (34") umfasst, die dazu angepasst ist, die Position des gesamten Implantats (1) gegen eine Gefäßwand zu sichern, und wobei die Kapsel (3) extern an der stentartigen Struktur (34") mittels eines oder mehrerer Befestigungselemente (35) befestigt ist.

**11.** System nach einem der Ansprüche 1-9, wobei das Implantat (1) planar ist und ein dielektrisches Substrat (29) aus einem flexiblen oder anpassungsfähigen Material umfasst, und wobei das Implantat (1) gegebenenfalls in Kontakt mit einer stentartigen Struktur (34") angeordnet ist, die dazu angepasst ist, die Position des gesamten Implantats (1) gegen eine Gefäßwand zu sichern.

**12.** System nach einem der Ansprüche 1-9, wobei das Implantat (1) eine langgestreckte, nicht starre Struktur umfasst, die mittels der stentartigen Struktur (34") aufgeweitet und an einer Gefäßwand gesichert werden kann, wobei das Implantat (1) ferner ein hohles Netzwerk von Verbindungen zwischen den Aufzeichnungs- und/oder Stimulationselektroden (11, 11') und der Kapsel (3) mittels Drähten (24) umfasst, die flexibel oder plastisch verformbar sind.

**13.** System nach einem der vorhergehenden Ansprüche, wobei:

- die Leistungstransferstufe (7) der elektronischen Schaltung (5) einen Blockierkondensator (7''') umfasst, der mit dem Volumenleitungselektrodenpaar (4, 4') oder der Vielzahl von Kommunikationselektroden in Reihe geschaltet ist, wobei der Blockierkondensator (7''') dazu angepasst ist, den Durchgang des Gleichstroms zu verhindern und den Fluss des Hochfrequenzstroms zu und von der elektronischen Schaltung (5) zu ermöglichen;
- die Kommunikationsstufe (8) eine Demodulatoreinheit (12) umfasst, die dazu angepasst ist, den Hochfrequenzstrom zu demodulieren; und
- die digitale Steuereinheit (6) mit einem Lastmodulator (17) verbunden ist, der dazu angepasst ist, die Last des Implantats (1) symmetrisch oder asymmetrisch zu modulieren.

**14.** System nach einem der vorhergehenden Ansprüche, wobei die externe Einheit (9) Folgendes umfasst:

- eine Stromversorgung (21);
- eine digitale Steuereinheit (6') und einen Modulator (22), dazu angepasst, Hochfrequenzsignale zu generieren, die dazu angepasst sind, ein oder mehrere Implantate (1) durch Volumenleitung zu versorgen und mit ihnen zu kommunizieren;
- Empfangsmittel, die dazu angepasst sind, das eine oder die mehreren Signale von dem einen oder den mehreren Implantaten (1) durch die Kopf- oder Halskörperregion (2) mittels Volumenleitung zu empfangen;
- Verarbeitungsmittel, die dazu angepasst sind, Informationen zu verarbeiten, die mit dem einen oder den mehreren Signalen assoziiert sind, die von dem einen oder den mehreren Implantaten (1) empfangen werden; und
- einen Signalverstärker (18'), der dazu angepasst ist, das eine oder die mehreren Verwaltungssignale, die von der digitalen Steuereinheit (6') und dem Modulator (22) generiert werden, zu verstärken; und die Signale durch Volumenleitung an ein oder mehrere Implantate (1) durch die Kopf- oder Halskörperregion (2) zuzuführen.

**15.** System nach einem der vorhergehenden Ansprüche, wobei die externe Einheit (9) eine Vielzahl von externen Elektroden (10, 10', 10', 10") und ein oder mehrere Schaltelemente (15) oder permanente Anschlüsse umfasst, die elektrisch mit der einen oder den mehreren externen Elektroden (10, 10', 10", 10''') verbunden sind, um selektiv einen Satz von ihnen auszuwählen, um den Hochfrequenzstrom zuzuführen und/oder selektiv eine oder mehrere von ihnen kurzzuschließen; und/oder mindestens eine externe Elektrode (10, 10'), die dazu angepasst ist, auf der Kopfhaut platziert zu werden; und mindestens eine externe Elektrode (10, 10'), die dazu angepasst ist, in der Halsregion platziert zu werden.

**Revendications**

1. Système d'alimentation ou de commande de l'électronique des implants de la tête ou du cou par conduction volumique, comprenant :

   - au moins un implant allongé (1), adapté pour être implanté à l'intérieur d'une région du corps (2), ladite région du corps (2) étant comprise dans la tête ou le cou d'un être humain ou d'un animal, dans lequel l'implant (1) comprend :

     a) une capsule hermétique (3) ;
     b) une pluralité d'électrodes primaires, comprenant une paire d'électrodes de conduction volumique (4, 4') ; lesdites électrodes primaires étant disposées, au moins en partie, à l'extérieur de la capsule (3) ;
     c) un circuit électronique (5) logé à l'intérieur de la capsule (3) et connecté électriquement aux électrodes primaires, comprenant :

       - une unité de commande numérique (6) ;
       - un étage de transfert de puissance (7) adapté pour recevoir au moins une partie de l'énergie d'un courant à haute fréquence transmis par conduction volumique à travers la paire d'électrodes de conduction volumique (4, 4'), et pour transférer la puissance associée à ladite au moins une partie de l'énergie du courant à haute fréquence à l'unité de commande numérique (6) et à un étage de communication (8) ;
       - un étage de communication (8) adapté pour détecter, à travers deux des électrodes primaires, le courant à haute fréquence transmis par conduction volumique à la paire d'électrodes de conduction volumique (4, 4') ; et pour traiter le courant à haute fréquence au moyen de l'unité de commande numérique (6) ; et
       - une unité externe (9) adaptée pour générer au moins le courant à haute fréquence afin de transférer la puissance à l'au moins un implant (1) et de communiquer avec lui ; dans lequel la fréquence fondamentale du courant à haute fréquence est comprise entre 1 MHz et 200 MHz ;

   dans lequel

   la capsule hermétique (3) de l'implant (1) est allongée et comprend un ou plusieurs segments diélectriques (3') entrecoupés d'un ou plusieurs segments conducteurs (3") ; et
   l'unité externe (9) est connecté électriquement à une pluralité d'électrodes externes (10, 10'), dans lequel au moins deux desdits électrodes externes (10, 10') comprennent des zones de contact correspondantes adaptées pour être disposées sur la région du corps (2), à des positions sensiblement opposées le long d'un axe longitudinal défini par l'implant allongé (1) ; et dans lequel la somme des zones de contact de chacune desdites électrodes externes (10, 10') est comprise entre 1 cm$^2$ et 250 cm$^2$.

2. Système selon la revendication précédente, dans lequel les électrodes primaires comprennent en outre une pluralité d'électrodes de communication ; dans lequel l'étage de communication (8) du circuit électronique (5) de l'implant (1) est en outre adapté pour échanger, par l'intermédiaire de deux des électrodes primaires, un ou plusieurs signaux de gestion avec l'unité externe (9) par conduction volumique.

3. Système selon l'une quelconque des revendications précédentes, dans lequel le circuit électronique (5) de l'implant (1) comprend en outre un étage d'acquisition de biopotentiels (13) comprenant des amplificateurs, des filtres passe-bande, des convertisseurs analogique-numérique et des éléments de commutation (15), ledit étage d'acquisition de biopotentiels (13) étant adapté pour enregistrer un ou plusieurs signaux électriques provenant de la région du corps à travers au moins la paire d'électrodes de conduction volumique (4, 4') et/ou une ou plusieurs électrodes d'enregistrement et de stimulation (11, 11'), les signaux électriques dépendant d'une différence de tension à l'extérieur de la capsule (3).

**4.** Système selon l'une quelconque des revendications précédentes, dans lequel le circuit électronique (5) de l'implant (1) comprend en outre un étage de neurostimulation (14) adapté pour générer et délivrer des signaux électriques à l'extérieur de l'implant (1) par l'intermédiaire de la paire d'électrodes de conduction volumique (4, 4') et/ou de l'une ou plusieurs électrodes d'enregistrement et de stimulation (11, 11'), ledit étage de neurostimulation (14) comprenant au moins l'un des éléments suivants : un générateur d'impulsions (10), des éléments de redressement (7') et des éléments de commutation (15).

**5.** Système selon l'une quelconque des revendications précédentes, dans lequel un ou plusieurs segments conducteurs (3") de la capsule hermétique (3) sont connectés électriquement à une ou plusieurs électrodes primaires (4, 4") de l'implant (1) ou à une ou plusieurs électrodes d'enregistrement et/ou de stimulation (11, 11') de l'implant (1), disposées à l'extérieur de la capsule (3), au moyen d'un ou plusieurs fils (24) ; ledit implant (1) comprenant un isolement non hermétique (25) qui loge, au moins en partie, la capsule (3) et les fils (24).

**6.** Système selon l'une quelconque des revendications précédentes, dans lequel la capsule hermétique (3) comprend en outre une tubulure interne (28) pour renforcer la structure et, éventuellement, pour loger les fils (24) qui connectent les segments conducteurs (3") au circuit électronique (5).

**7.** Système selon l'une quelconque des revendications précédentes, dans lequel les segments conducteurs (3") n'entourent pas la totalité de la section transversale de la capsule (3).

**8.** Système selon l'une quelconque des revendications précédentes, dans lequel la capsule (3) comprend des segments étagés dans lesquels des segments diélectriques (3') sont intercalés avec des segments conducteurs (3").

**9.** Système selon l'une quelconque des revendications précédentes, dans lequel l'implant (1) a la forme d'un dispositif semi-flexible filiforme et dans lequel la capsule hermétique (3) est contenue dans un corps flexible non hermétique (32) en matériau isolant qui contient également les fils (24) pour connecter la capsule (3) à une ou plusieurs électrodes primaires (4, 4") de l'implant (1) ou à une ou plusieurs électrodes d'enregistrement et/ou de stimulation (11, 11') de l'implant (1).

**10.** Système selon l'une quelconque des revendications précédentes, dans lequel l'implant (1) comprend une structure de type stent (34") adaptée pour sécuriser la position de l'ensemble de l'implant (1) contre la paroi d'un vaisseau, et dans lequel la capsule (3) est fixée extérieurement à la structure de type stent (34") au moyen d'un ou de plusieurs éléments de fixation (35).

**11.** Système selon l'une quelconque des revendications 1 à 9, dans lequel l'implant (1) est plan et comprend un substrat diélectrique (29) constitué d'un matériau flexible ou conformable, et dans lequel l'implant (1) est éventuellement disposé en contact avec une structure de type stent (34") adaptée pour sécuriser la position de l'ensemble de l'implant (1) contre la paroi d'un vaisseau.

**12.** Système selon l'une quelconque des revendications 1 à 9, dans lequel l'implant (1) comprend une structure allongée non rigide qui peut être étendue et fixée à la paroi d'un vaisseau au moyen de la structure de type stent (34"), dans lequel l'implant (1) comprend en outre un réseau creux de connexions entre les électrodes d'enregistrement et/ou de stimulation (11, 11') et la capsule (3) au moyen de fils (24) qui sont flexibles ou plastiquement déformables.

**13.** Système selon l'une quelconque des revendications précédentes, dans lequel :

- l'étage de transfert de puissance (7) du circuit électronique (5) comprend un condensateur de blocage (7"') connecté en série avec la paire d'électrodes de conduction volumique (4, 4') ou la pluralité d'électrodes de communication, ledit condensateur de blocage (7"') étant adapté pour empêcher le passage du courant cc et pour permettre le flux du courant à haute fréquence vers et à partir du circuit électronique (5) ;
- l'étage de communication (8) comprend une unité de démodulation (12) adaptée pour démoduler le courant à haute fréquence ; et
- l'unité de commande numérique (6) est connectée à un modulateur de charge (17) adapté pour moduler symétriquement ou asymétriquement la charge de l'implant (1).

**14.** Système selon l'une quelconque des revendications précédentes, dans lequel l'unité externe (9) comprend :

- une alimentation électrique (21) ;

- une unité de commande numérique (6') et un modulateur (22) adaptés pour générer des signaux à haute fréquence adaptés pour alimenter un ou plusieurs implants (1) et pour communiquer avec eux par conduction volumique ;
- des moyens de réception adaptés pour recevoir l'un ou plusieurs signaux provenant de l'un ou plusieurs implants (1) à travers la région du corps de la tête ou du cou (2) par conduction volumique ;
- des moyens de traitement adaptés pour traiter les informations associées à l'un ou plusieurs signaux reçus à partir de l'un ou plusieurs implants (1) ; et
- un amplificateur de signaux (18') adapté pour amplifier l'un ou plusieurs signaux de gestion générés par l'unité de commande numérique (6') et le modulateur (22) ; et pour délivrer lesdits signaux à un ou plusieurs implants (1) à travers ladite région du corps de la tête ou du cou (2) par conduction volumique.

**15.** Système selon l'une quelconque des revendications précédentes, dans lequel l'unité externe (9) comprend une pluralité d'électrodes externes (10, 10', 10', 10"), et un ou plusieurs éléments de commutation (15) ou interconnexions permanentes connectés électriquement à l'une ou plusieurs électrodes externes (10, 10', 10", 10‴) pour sélectionner sélectivement un ensemble d'entre elles pour délivrer le courant à haute fréquence et/ou pour court-circuiter sélectivement une ou plusieurs d'entre elles ; et/ou au moins une électrode externe (10, 10') adaptée pour être placée sur le cuir chevelu ; et au moins une électrode externe (10, 10') adaptée pour être placée dans la région du cou.

**FIG. 1**

**FIG. 2**

**FIG. 3**

**FIG. 4**

**FIG. 5**

A)

B)

**FIG. 6**

**FIG. 7**

**FIG. 8**

**FIG. 9**

**FIG. 10**

**FIG. 11**

**FIG. 12**

FIG. 13

FIG. 14

FIG. 15

**FIG. 16**

**FIG. 17**

**FIG. 18**

FIG. 19

FIG. 20

**FIG. 21**

**FIG. 22**

**FIG. 23**

**FIG. 24**

**FIG. 25**

**FIG. 26**

**FIG. 27**

**FIG. 28**

**FIG. 29**

**FIG. 30**

**FIG. 31**

**FIG. 32**

A)

33

4, 4'

B)

**FIG. 33**

**FIG. 34**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10729530 B2

### Non-patent literature cited in the description

- **KRAUSS et al.** Technology of deep brain stimulation: current status and future directions. *Nature Reviews Neurology,* 2020, 1-13
- **S.E. JOHN et al.** The future potential of the Stentrode. *Expert review of medical devices,* 2019, vol. 16 (10), 841-843
- **T. J. OXLEY et al.** Minimally invasive endovascular stent-electrode array for high-fidelity, chronic recordings of cortical neural activity. *Nature biotechnology,* 2016, vol. 34 (3), 320-327
- **G. L. BARBRUNI et al.** Miniaturised Wireless Power Transfer Systems for Neurostimulation: A Review. *IEEE Transactions on Biomedical Circuits and Systems,* 2020
- **J. MINGUILLÓN et al.** Powering electronic implants by high-frequency volume conduction: in human validation. *bioRxiv,* 2021
- **L. BECERRA-FAJARDO et al.** Demonstration of 2-mm thick microcontrolled injectable stimulators based on rectification of high frequency current bursts. *IEEE Transactions on Neural Systems and Rehabilitation Engineering,* August 2017, vol. 25 (8), 1343-1352
- **M. GOSSELIN et al.** Development of a new generation of high-resolution anatomical models for medical device evaluation: the Virtual Population 3.0. *Physics in medicine and biology,* 2014, vol. 59 (18), 5287-303
- **M. TUDELA-PI et al.** Powering implants by galvanic coupling: A validated analytical model predicts powers above 1 mW in injectable implants. *IFMBE Proceedings,* 2019, vol. 68 (3